# EUROPEAN PATENT APPLICATION

(11) **EP 4 653 431 A1**
(43) Date of publication of application: **26.11.2025**
(21) Application number: 24744332.8
(22) Date of filing: 18.01.2024
(51) Int. Cl.: C07D 403/12, A61K 31/4985, A61P 35/00

(54) **PYRAZOLE DERIVATIVE, AND PHARMACEUTICALLY ACCEPTABLE SALT, STEREOISOMER, PHARMACEUTICAL COMPOSITION AND USE THEREOF**

(30) Priority: 19.01.2023 CN 202310059693
(71) Applicant: Starg (Wuhan) Pharmaceutical Technology Co., Ltd, Wuhan, Hubei 430000 (CN)
(72) Inventor: HU, Pu, Wuhan, Hubei 430000 (CN); LU, Juquan, Wuhan, Hubei 430000 (CN)
(74) Representative: J A Kemp LLP
(86) International application number: PCT/CN2024/072893
(87) International publication number: WO 2024/153158

(57) **Abstract**

A pyrazole derivative having a structure as shown in formula (I), and a pharmaceutically acceptable salt, stereoisomer, pharmaceutical composition and the use thereof. The compound has a good inhibitory activity and selectivity on CDK2.

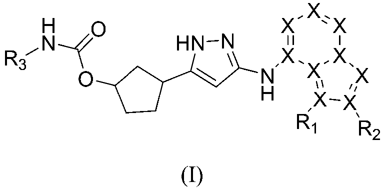

## Description

This application claims priority to:
2023100596930, filed on January 19, 2023.

### TECHNICAL FIELD

The present application relates to the field of pharmaceutical technology, specifically relating to a pyrazole derivative, a pharmaceutically acceptable salt thereof, a stereoisomer, a pharmaceutical composition, and use thereof.

### BACKGROUND

Cyclin-dependent kinases (CDKs) belong to the serine/threonine kinase family. They are involved in physiological processes such as cell proliferation and transcription. Based on functional differences, CDKs can be categorized into two groups: one group of CDKs participates in cell cycle regulation, primarily including CDK1, CDK2, CDK4, CDK6, etc.; the other group of CDKs participates in transcription regulation, primarily including CDK7, CDK8, CDK9, CDK12, CDK13, etc.

The dysregulation of CDK2 activity is often observed in various human cancers, thus gaining significant interest from researchers. CDK2 plays a critical role in promoting the G1/S transition and S-phase progression. CDK2 forms a complex with cyclin E and phosphorylates members of the retinoblastoma family (such as pRb), leading to the release and activation of E2F transcription factors, driving the cell cycle transition from G1 phase to S phase, subsequently activating CDK2/cyclin A, thereby facilitating processes such as DNA synthesis and replication during the cell cycle.

Increased copy number and overexpression of cyclin E1 have been identified in ovarian cancer, gastric cancer, endometrial cancer, breast cancer, and other cancers, and are positively correlated with poor prognosis of the corresponding tumors. In ER+ breast cancer cells, high expression of cyclin E2 is often accompanied by resistance to hormonal therapy, and amplification or overexpression of cyclin E is closely linked to poor prognosis in breast cancer. In HER2+ breast cancer, amplification of cyclin E has been reported to contribute to resistance to trastuzumab. Furthermore, reports indicate that cyclin E overexpression plays a significant role in the progression of triple-negative breast cancer or inflammatory breast cancer. Consequently, CDK2 may serve as a crucial anti-tumor target.

### SUMMARY

In view of the above, the present application provides a pyrazole derivative with high selectivity for inhibiting the activity of CDK2, a pharmaceutically acceptable salt thereof, or a stereoisomer thereof.

The present invention is achieved through the following technical solutions.

A pyrazole derivative represented by Formula (I), or a pharmaceutically acceptable salt thereof, or a stereoisomer thereof: 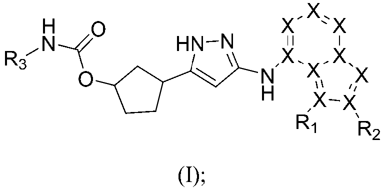 wherein:
X, at each occurrence, is independently selected from N or CR₄; R₄ is absent or selected from -H, -D, -F, -Cl, -Br, -I, linear alkyl having 1 to 6 carbon atoms, deuterated linear alkyl having 1 to 6 carbon atoms, branched alkyl having 3 to 6 carbon atoms, or an aromatic heterocyclic group;
R₁ is selected from -H, -D, -F, -Cl, -Br, -I, linear alkyl having 1 to 20 carbon atoms, deuterated linear alkyl having 1 to 20 carbon atoms, branched alkyl having 3 to 20 carbon atoms, deuterated branched alkyl having 3 to 20 carbon atoms, cycloalkyl having 3 to 20 carbon atoms, deuterated cycloalkyl having 3 to 20 carbon atoms, linear alkoxy having 1 to 20 carbon atoms, deuterated linear alkoxy having 1 to 20 carbon atoms, branched alkoxy having 3 to 20 carbon atoms, deuterated branched alkoxy having 3 to 20 carbon atoms, cycloalkoxy having 3 to 20 carbon atoms, or deuterated cycloalkoxy having 3 to 20 carbon atoms;
R₃ is selected from substituted or unsubstituted alkyl having 1 to 6 carbon atoms, substituted or unsubstituted fluoroalkyl having 1 to 6 carbon atoms, substituted or unsubstituted cycloalkyl having 3 to 7 carbon atoms, or a substituted or unsubstituted heterocyclic group having 4 to 7 ring atoms;
R₂ is selected from -F, -Cl, -OH, -CN, -NR₅R₆, -CH₂-ONR₇R₈, -CH₂-ON=R₉, -CH₂N(R₁₀)OR₁₁, substituted or unsubstituted alkyl having 1 to 4 carbon atoms, substituted or unsubstituted fluoroalkyl having 1 to 4 carbon atoms, substituted or unsubstituted alkoxy having 1 to 4 carbon atoms, substituted or unsubstituted fluoroalkoxy having 1 to 4 carbon atoms, or substituted or unsubstituted cycloalkyl having 3 to 8 carbon atoms;
R₅, R₆, R₇, R₈, R₁₀, and R₁₁ are each independently selected from -H, -D, linear alkyl having 1 to 20 carbon atoms, deuterated linear alkyl having 1 to 20 carbon atoms, branched alkyl having 3 to 20 carbon atoms, deuterated branched alkyl having 3 to 20 carbon atoms, cycloalkyl having 3 to 20 carbon atoms, deuterated cycloalkyl having 3 to 20 carbon atoms, linear alkoxy having 1 to 20 carbon atoms, deuterated linear alkoxy having 1 to 20 carbon atoms, branched alkoxy having 3 to 20 carbon atoms, deuterated branched alkoxy having 3 to 20 carbon atoms, cycloalkoxy having 3 to 20 carbon atoms, or deuterated cycloalkoxy having 3 to 20 carbon atoms;
R₉ is selected from linear alkylidene having 1 to 20 carbon atoms, deuterated linear alkylidene having 1 to 20 carbon atoms, branched alkylidene having 3 to 20 carbon atoms, deuterated branched alkylidene having 3 to 20 carbon atoms, cycloalkylidene having 3 to 20 carbon atoms, deuterated cycloalkylidene having 3 to 20 carbon atoms, or combinations thereof.

In an embodiment, the pyrazole derivative is represented by Formula (II):

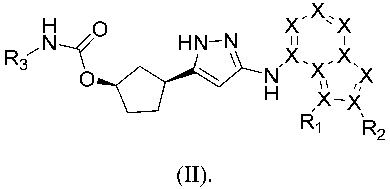

In an embodiment, the pyrazole derivative is represented by Formula (III):

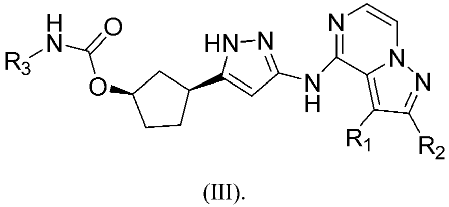

In an embodiment, R₁ is selected from -H, -D, -F, -Cl, -Br, -I, linear alkyl having 1 to 10 carbon atoms, deuterated linear alkyl having 1 to 10 carbon atoms, branched alkyl having 3 to 10 carbon atoms, deuterated branched alkyl having 3 to 10 carbon atoms, cycloalkyl having 3 to 10 carbon atoms, deuterated cycloalkyl having 3 to 10 carbon atoms, or combinations thereof.

In an embodiment, R₁ is selected from -H, -D, -F, -Cl, -Br, linear alkyl having 1 to 6 carbon atoms, deuterated linear alkyl having 1 to 6 carbon atoms, branched alkyl having 3 to 6 carbon atoms, or deuterated branched alkyl having 3 to 6 carbon atoms.

In an embodiment, R₁ is selected from -H, -D, -F, -Cl, -Br, linear alkyl having 1 to 3 carbon atoms, deuterated linear alkyl having 1 to 3 carbon atoms, branched alkyl having 3 to 6 carbon atoms, or deuterated branched alkyl having 3 to 6 carbon atoms.

In an embodiment, R₁ is selected from -H, -D, -F, -CH₃, -CH₂CH₃, -CH₂CH₂CH₃, and -CH(CH₃)₂.

In an embodiment, R₂ is selected from -F, -Cl, -OH, -CN, -NR₅R₆, -CH₂-ONR₇R₈, -CH₂-ON=R₉, -CH₂N(R₁₀)OR₁₁, substituted or unsubstituted alkyl having 1 to 4 carbon atoms, substituted or unsubstituted fluoroalkyl having 1 to 4 carbon atoms, substituted or unsubstituted alkoxy having 1 to 4 carbon atoms, substituted or unsubstituted fluoroalkoxy having 1 to 4 carbon atoms, or substituted or unsubstituted cycloalkyl having 3 to 8 carbon atoms.

In an embodiment, R₃ is selected from substituted or unsubstituted alkyl having 1 to 3 carbon atoms, substituted or unsubstituted fluoroalkyl having 1 to 3 carbon atoms, substituted or unsubstituted cycloalkyl having 3 to 7 carbon atoms, or a substituted or unsubstituted heterocyclic group having 4 to 7 ring atoms.

In an embodiment, R₃ is selected from substituted or unsubstituted -CH₃, substituted or unsubstituted -CH₂CH₃, substituted or unsubstituted -CH₂CH₂CH₃, or substituted or unsubstituted -CH(CH₃)₂.

In an embodiment, R₃ is selected from substituted or unsubstituted -CH(CH₃)₂. In an embodiment, R₂ is selected from -F, -Cl, -OH, -CN, -NR₅R₆, -CH₂-ONR₇R₈, -CH₂-ON=R₉, -CH₂N(R₁₀)OR₁₁, substituted or unsubstituted -CH₃, substituted or unsubstituted -CH₂CH₃, substituted or unsubstituted -CH₂CH₂CH₃, substituted or unsubstituted -CH(CH₃)₂, substituted or unsubstituted fluorinated -CH₃, substituted or unsubstituted fluorinated -CH₂CH₃, substituted or unsubstituted fluorinated -CH₂CH₂CH₃, or substituted or unsubstituted fluorinated -CH(CH₃)₂.

In an embodiment, R₅, R₆, R₇, R₈, R₁₀, and R₁₁ are each independently selected from -H, -D, linear alkyl having 1 to 10 carbon atoms, deuterated linear alkyl having 1 to 10 carbon atoms, branched alkyl having 3 to 10 carbon atoms, deuterated branched alkyl having 3 to 10 carbon atoms, cycloalkyl having 3 to 10 carbon atoms, deuterated cycloalkyl having 3 to 10 carbon atoms, linear alkoxy having 1 to 10 carbon atoms, deuterated linear alkoxy having 1 to 10 carbon atoms, branched alkoxy having 3 to 10 carbon atoms, deuterated branched alkoxy having 3 to 10 carbon atoms, cycloalkoxy having 3 to 10 carbon atoms, or deuterated cycloalkoxy having 3 to 10 carbon atoms.

In an embodiment, R₅, R₆, R₇, R₈, R₁₀, and R₁₁ are each independently selected from -H, -D, linear alkyl having 1 to 6 carbon atoms, deuterated linear alkyl having 1 to 6 carbon atoms, branched alkyl having 3 to 6 carbon atoms, deuterated branched alkyl having 3 to 6 carbon atoms, cycloalkyl having 3 to 6 carbon atoms, deuterated cycloalkyl having 3 to 6 carbon atoms, linear alkoxy having 1 to 6 carbon atoms, deuterated linear alkoxy having 1 to 6 carbon atoms, branched alkoxy having 3 to 6 carbon atoms, deuterated branched alkoxy having 3 to 6 carbon atoms, cycloalkoxy having 3 to 6 carbon atoms, or deuterated cycloalkoxy having 3 to 6 carbon atoms.

In an embodiment, R₅, R₆, R₇, R₈, R₁₀, and R₁₁ are each independently selected from -H, -D, linear alkyl having 1 to 3 carbon atoms, deuterated linear alkyl having 1 to 3 carbon atoms, branched alkyl having 3 to 6 carbon atoms, deuterated branched alkyl having 3 to 6 carbon atoms, cycloalkyl having 3 to 6 carbon atoms, deuterated cycloalkyl having 3 to 6 carbon atoms, linear alkoxy having 1 to 3 carbon atoms, deuterated linear alkoxy having 1 to 3 carbon atoms, branched alkoxy having 3 to 6 carbon atoms, deuterated branched alkoxy having 3 to 6 carbon atoms, cycloalkoxy having 3 to 6 carbon atoms, or deuterated cycloalkoxy having 3 to 6 carbon atoms.

In an embodiment, R₅, R₆, R₇, R₈, R₁₀, and R₁₁ are each independently selected from -H, -D, -CH₃, -CH₂CH₃, -CH₂CH₂CH₃, and -CH(CH₃)₂.

In an embodiment, R₉ is selected from linear alkylidene having 1 to 20 carbon atoms, deuterated linear alkylidene having 1 to 20 carbon atoms, branched alkylidene having 3 to 20 carbon atoms, deuterated branched alkylidene having 3 to 20 carbon atoms, cycloalkylidene having 3 to 20 carbon atoms, or deuterated cycloalkylidene having 3 to 20 carbon atoms.

In an embodiment, R₉ is selected from linear alkylidene having 1 to 10 carbon atoms, deuterated linear alkylidene having 1 to 10 carbon atoms, branched alkylidene having 3 to 10 carbon atoms, deuterated branched alkylidene having 3 to 10 carbon atoms, cycloalkylidene having 3 to 10 carbon atoms, or deuterated cycloalkylidene having 3 to 10 carbon atoms.

In an embodiment, R₉ is selected from linear alkylidene having 1 to 6 carbon atoms, deuterated linear alkylidene having 1 to 6 carbon atoms, branched alkylidene having 3 to 6 carbon atoms, deuterated branched alkylidene having 3 to 6 carbon atoms, cycloalkylidene having 3 to 6 carbon atoms, or deuterated cycloalkylidene having 3 to 6 carbon atoms.

In an embodiment, R₉ is selected from linear alkylidene having 1 to 3 carbon atoms, deuterated linear alkylidene having 1 to 3 carbon atoms, branched alkylidene having 3 to 6 carbon atoms, deuterated branched alkylidene having 3 to 6 carbon atoms, cycloalkylidene having 3 to 6 carbon atoms, or deuterated cycloalkylidene having 3 to 6 carbon atoms.

In an embodiment, R₉ is selected from =CH₂, =CHCH₃, =CHCH₂CH₃, and =C (CH₃)₂.

In an embodiment, R₂ is any one selected from the following structures:

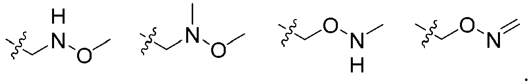

The present application provides following compounds or their pharmaceutically acceptable salts:

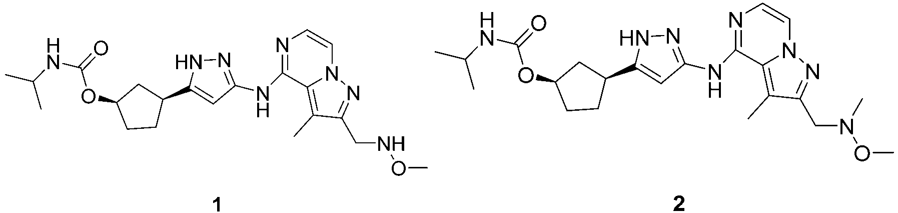

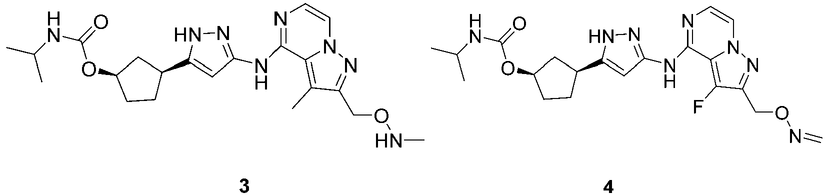

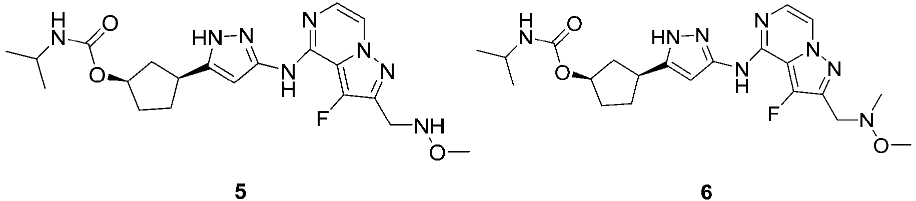

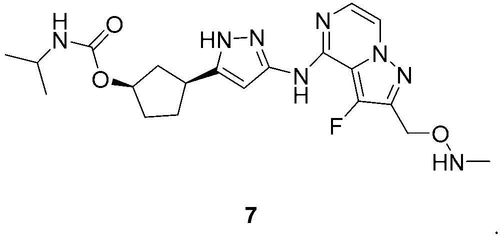

In a specific embodiment, the pharmaceutically acceptable salt is a salt of an alkyl acid; further, the pharmaceutically acceptable salt is a formate salt.

In a specific embodiment, the pharmaceutically acceptable salt is a formate salt or a hydrochloride salt.

The present application further provides the use of the above-described pyrazole derivative, or its pharmaceutically acceptable salt, or its stereoisomer in preparation of a drug for treating and/or preventing a disease related to or mediated by CDK2 activity.

In a specific embodiment, the disease related to or mediated by CDK2 activity is cancer.

In a specific embodiment, the cancer is one or more of ovarian cancer, gastric cancer, endometrial cancer, or breast cancer.

The present application further provides a pharmaceutical composition including the above-described pyrazole derivative, or its pharmaceutically acceptable salt, or its stereoisomer, and a pharmaceutically acceptable carrier.

It can be understood that, within the scope of the present application, the above technical features of the present application and the technical features specifically described below (e.g., in the examples) can be combined with each other to form new or preferred technical solutions. Due to space limitations, they are not exhaustively listed herein.

Compared with the prior art, the pyrazole derivative, or its pharmaceutically acceptable salt, or its stereoisomer of the present application has the following beneficial effects:
Through long-term and in-depth research, the inventors have unexpectedly discovered a novel pyrazole derivative. The pyrazole derivative of the present application exhibits surprising inhibitory activity and selectivity against CDK2, and can be used for the treatment of various cancers with high cyclin E expression, particularly demonstrating excellent therapeutic effects in cancer patients resistant to CDK4/6 inhibitors.

### DETAILED DESCRIPTION

In order to facilitate the understanding of the present application, the present application will be comprehensively described in details with reference to the embodiments. The preferred implementations of the present application are provided in the embodiments. However, the present application can be implemented in many different forms and therefore is not limited to the embodiments described herein. On the contrary, the purpose of providing these embodiments is to make the understanding of the present application more thorough and comprehensive.

### Definition of Terms

Unless otherwise specified, the following terms and phrases used herein are intended to have the meanings set forth below. A specific term or phrase, in the absence of a particular definition, should not be considered indefinite or unclear, but rather should be understood according to its ordinary meaning. When a trade name appears herein, it is intended to refer to the corresponding product or its active ingredient.

The term "pharmaceutically acceptable" refers to those compounds, materials, compositions, and/or dosage forms that, within the scope of sound medical judgment, are suitable for contact with the tissues of humans and animals without excessive toxicity, irritation, allergic response, or other problems or complications, commensurate with a reasonable benefit-risk ratio.

The term "pharmaceutically acceptable salt" refers to a salt of a compound of the present application, prepared from the compound discovered in the present application having a specific substituent and a relatively non-toxic acid or base. When the compound of the present application contains a relatively acidic functional group, a base addition salt can be obtained by contacting such a compound with a sufficient amount of a base in a pure solution or a suitable inert solvent. When the compound of the present application contains a relatively basic functional group, an acid addition salt can be obtained by contacting such a compound with a sufficient amount of an acid in a pure solution or a suitable inert solvent. Examples of the pharmaceutically acceptable acid addition salt include inorganic acid salts and organic acid salts, as well as salts of amino acids (such as arginine) and salts of organic acids such as glucuronic acid. Certain specific compounds of the present application contain both basic and acidic functional groups, allowing them to be converted into either base or acid addition salts.

The pharmaceutically acceptable salt of the present application can be synthesized from a parent compound containing an acidic or basic group by a conventional chemical method. Generally, such a salt is prepared by reacting the compound in free acid or base form with a stoichiometric amount of an appropriate base or acid in water, an organic solvent, or a mixture of both.

The compounds of the present application may have specific geometric or stereoisomeric forms. The present application contemplates all such compounds, including cis and trans isomers, (-)- and (+)-enantiomers, (R)- and (S)-enantiomers, diastereomers, (D)-isomers, (L)-isomers, as well as their racemic mixtures and other mixtures, such as enantiomerically or diastereomerically enriched mixtures, all of which are within the scope of the present application. Additional asymmetric carbon atoms may be present in substituents such as alkyl groups. All such isomers, as well as their mixtures, are included within the scope of the present application.

Unless otherwise specified, the term "enantiomers" or "optical isomers" refers to stereoisomers that are mirror images of each other.

Unless otherwise specified, the term "cis-trans isomers" or "geometric isomers" refers to isomers arising from the restricted rotation of a double bond or a single bond of ring-forming carbon atoms.

Unless otherwise specified, the term "diastereomers" refers to stereoisomers where molecules have two or more chiral centers and are non-mirror images of each other.

Unless otherwise specified, "(+)" denotes dextrorotatory, "(-)" denotes levorotatory, and "(±)" denotes racemic.

Unless otherwise specified, the absolute configuration of a stereocenter is represented with a wedge-shaped solid bond ( 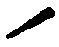 ) and a wedge-shaped dashed bond ( 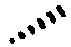 ), the relative configuration of a stereocenter is represented with a straight solid bond ( 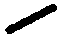 ) and a straight dashed bond ( 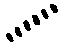 ); a wavy line ( 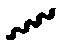 ) represents either a wedge-shaped solid bond ( 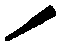 ) or a wedge-shaped dashed bond ( 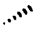 ), or a wavy line ( 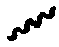 ) represents either a straight solid bond ( 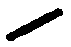 ) or a straight dashed bond ( 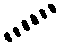 ).

Unless otherwise specified, the term "tautomers" or "tautomeric form" refers to isomers with different functional groups that are in dynamic equilibrium at room temperature and can rapidly interconvert. For example, in the present application, 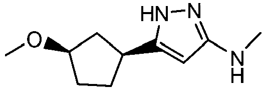 and 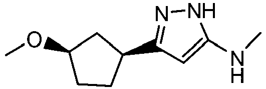 are tautomers. If tautomerism is possible (e.g., in solution), a chemical equilibrium of the tautomers can be achieved. For instance, proton tautomers (also known as prototropic tautomers) achieve interconversions via proton migration, such as keto-enol tautomerism and imine-enamine tautomerism. Valence tautomers involve interconversions through the reorganization of bonding electrons. A specific example of keto-enol tautomerism is the interconversion between the tautomers pentane-2,4-dione and 4-hydroxypent-3-en-2-one.

Unless otherwise specified, the terms "enriched in one isomer," "isomer-enriched," "enriched in one enantiomer," or "enantiomer-enriched" refer to the content of one isomer or enantiomer is less than 100%, and the content of that isomer or enantiomer is greater than or equal to 60%, or greater than or equal to 70%, or greater than or equal to 80%, or greater than or equal to 90%, or greater than or equal to 95%, or greater than or equal to 96%, or greater than or equal to 97%, or greater than or equal to 98%, or greater than or equal to 99%, or greater than or equal to 99.5%, or greater than or equal to 99.6%, or greater than or equal to 99.7%, or greater than or equal to 99.8%, or greater than or equal to 99.9%.

Unless otherwise specified, the term "isomeric excess" or "enantiomeric excess" refers to the difference between the relative percentages of two isomers or two enantiomers. For example, where the content of one isomer or enantiomer is 90% and the content of the other isomer or enantiomer is 10%, the isomeric or enantiomeric excess (ee value) is 80%.

Optically active (R)- and (S)-isomers, as well as *D-* and L-isomers, can be prepared by chiral synthesis, chiral reagents, or other conventional techniques. If a specific enantiomer of a compound of the present application is desired, it can be prepared by asymmetric synthesis or by derivatization with a chiral auxiliary, where the resulting diastereomeric mixture is separated, and the auxiliary group is cleaved to provide the pure desired enantiomer. Alternatively, when a molecule contains a basic functional group (such as an amino group) or an acidic functional group (such as a carboxyl group), a diastereomeric salt can be formed with an appropriate optically active acid or base, followed by diastereomeric resolution using conventional methods known in the art, and subsequent recovery of the pure enantiomer. Additionally, the separation of enantiomers and diastereomers is typically achieved by chromatography using a chiral stationary phase, optionally combined with chemical derivatization (e.g., forming carbamates from amines).

A compound of the present application may contain unnatural proportions of atomic isotopes at one or more of the atoms constituting the compound. For example, the compound may be labeled with radioactive isotopes such as tritium (³H), iodine-125 (¹²⁵I), or carbon-14 (¹⁴C). Also, for example, hydrogen may be substituted with deuterium to form a deuterated drug, where the bond between deuterium and carbon is stronger than that between ordinary hydrogen and carbon. Compared to non-deuterated drugs, deuterated drugs offer advantages such as reduced toxic side effects, increased drug stability, enhanced efficacy, and prolonged biological half-life. All isotopic variations of the compounds of the present application, whether radioactive or not, are encompassed within the scope of the present application.

The term "optional" or "optionally" refers to an event or condition described subsequently that may, but is not required to, occur, and the description includes instances where the event or condition occurs as well as instances where the event or condition does not occur.

The term "substituted" means that any one or more hydrogen atoms on a specific atom are replaced with a substituent. The substituents can include deuterium and hydrogen variants, provided that the valence of the specific atom is normal and the substituted compound is stable. The type and number of substituents can be arbitrary as long as they are chemically feasible. When the substituent is oxygen (i.e., =O), it means that two hydrogen atoms are replaced. The oxygen substitution does not occur on aromatic groups.

When any variable (e.g., R₁) appears more than once in the composition or structure of a compound, its definition in each instance is independent. Thus, for example, if a group is substituted with 0 to 2 R₁, the group can optionally be substituted with up to two R₁, and R₁ in each instance has independent options. Furthermore, a combination of substituents and/or their variants is permissible only if such a combination result in a stable compound.

When the number of a linking group is 0, such as -(CRR)₀-, it means that the linking group is a single bond.

When a variable is selected from a single bond, it means that the two groups the variable connects are directly linked. For example, when L in A-L-Z represents a single bond, the structure is actually A-Z.

When a substituent is vacant, it means that the substituent is absent. For example, when X in A-X is vacant, the structure is actually A. When a listed substituent does not indicate the specific atom through which it is attached to the substituted group, the substituent can be bonded via any of its constituent atoms. For example, a pyridyl group as a substituent can be connected to the substituted group through any carbon atom of the pyridine ring.

When a listed linking group does not indicate its direction of connection, the connection direction is arbitrary. For example, in the structure 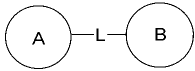 where the linking group L is -M-W-, -M-W- can connect ring A and ring B in the direction same as the reading order from left to right, forming 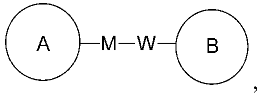 or in the direction opposite to the reading order from left to right, forming 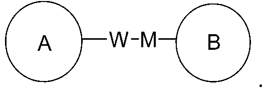 A combination of a linking group and a substituent and/or its variant is permissible only if such a combination results in a stable compound.

Unless otherwise specified, when a group has one or more connectable sites, any one or more of these sites can be connected to other groups through chemical bonds. When the chemical bonds are connected in a non-positional manner and there are H atoms at the connectable sites, the number of H atoms at the sites will correspondingly decrease with the number of the connected chemical bonds, resulting in a group with corresponding valence. The chemical bonds connecting the sites to other groups can be represented by straight solid bonds ( 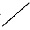 ), straight dashed bonds ( 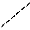 ), or wavy lines 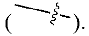 For example, the straight solid bond in -OCH₃ represents the connection to another group through the oxygen atom of the group; the straight dashed bonds in 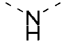 represent the connection to other groups through two ends of the nitrogen atom of the group; the wavy lines in 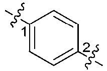 represent the connection to other groups through the carbon atoms at positions 1 and 2 of the phenyl group; 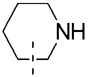 represents that any connectable site on the piperidinyl group can be connected to another group through one chemical bond, including at least the four connection ways 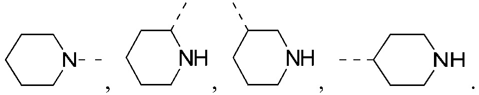 Even if a hydrogen atom is depicted on the -N-, 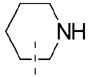 still includes 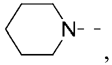 but when a chemical bond is formed, one hydrogen atom at that site will be removed, resulting in the corresponding monovalent piperidinyl group.

Unless otherwise specified, the number of atoms in a ring is typically defined as the number of ring members. For example, a "3- to 7-membered ring" refers to a "ring" composed of 3 to 7 atoms arranged in a cyclic manner.

Unless otherwise specified, the term "C₁₋₆ alkyl" represents to a saturated hydrocarbon group, either linear or branched, consisting of 1 to 6 carbon atoms, and preferably is C₁₋₄ alkyl, which can be monovalent (e.g., methyl), divalent (e.g., methylene), or polyvalent (e.g., methine). Examples of C₁₋₃ alkyl include, but are not limited to, methyl (Me), ethyl (Et), propyl (including n-propyl and isopropyl), and butyl (including n-butyl, isobutyl, t-butyl, and s-butyl).

Unless otherwise specified, the term "C₁₋₆ alkoxy" refers to an alkyl group containing 1 to 6 carbon atoms that is attached to the rest of the molecule through an oxygen atom, and preferably, is a C₁₋₃ alkoxy. Examples of C₁₋₃ alkoxy include, but are not limited to, methoxy, ethoxy, and propoxy (including n-propoxy and isopropoxy).

Unless otherwise specified, the term "C₁₋₆ alkylamino" refers to an alkyl group containing 1 to 6 carbon atoms that is attached to the rest of the molecule through an amino group, and preferably, is a C₁₋₃ alkylamino. Examples of C₁₋₃ alkylamino include, but are not limited to, -NHCH₃, -N(CH₃)₂, -NHCH₂CH₃, -N(CH₃)CH₂CH₃, -NHCH₂CH₂CH₃, -NHCH₂(CH₃)₂, etc.

Unless otherwise specified, the term "halogen" or "halo", either alone or as part of another substituent, refers to a fluorine, chlorine, bromine, or iodine atom.

Unless otherwise specified, the terms "5-membered heteroaryl ring" and "5-membered heteroaryl" are used interchangeably in the present application. The term "5-membered heteroaryl" refers to a monocyclic group with a conjugated π-electron system composed of 5 ring atoms, of which 1, 2, 3, or 4 ring atoms are heteroatoms independently selected from O, S, and N, with the remaining atoms being carbon. The nitrogen atoms may optionally be quaternized, and the nitrogen and sulfur heteroatoms may optionally be oxidized (i.e., NO and S(O)ₚ, where p is 1 or 2). The 5-membered heteroaryl can be connected to the rest of the molecule through a heteroatom or a carbon atom. Examples of 5-membered heteroaryl include, but are not limited to, pyrrolyl (including N-pyrrolyl, 2-pyrrolyl, 3-pyrrolyl, etc.), pyrazolyl (including 2-pyrazolyl, 3-pyrazolyl, etc.), imidazolyl (including N-imidazolyl, 2-imidazolyl, 4-imidazolyl, 5-imidazolyl, etc.), oxazolyl (including 2-oxazolyl, 4-oxazolyl, 5-oxazolyl, etc.), triazolyl (including 1H-1,2,3-triazolyl, 2H-1,2,3-triazolyl, 1H-1,2,4-triazolyl, 4H-1,2,4-triazolyl, etc.), tetrazolyl, isoxazolyl (3-isoxazolyl, 4-isoxazolyl, 5-isoxazolyl, etc.), thiazolyl (including 2-thiazolyl, 4-thiazolyl, 5-thiazolyl, etc.), furanyl (including 2-furanyl, 3-furanyl, etc.), and thienyl (including 2-thienyl, 3-thienyl, etc.).

Unless otherwise specified, Cₙ₋ₙ₊ₘ or Cₙ-Cₙ₊ₘ includes any specific case of n to n+m carbon atoms (e.g., examples of C₁₋₁₂ include C₁, C₂, C₃, C₄, C₅, C₆, C₇, C₈, C₉, C₁₀, C₁₁, and C₁₂), as well as any range within n to n+m (e.g., examples of C₁₋₁₂ include C₁₋₃, C₁₋₆, C₁₋₉, C₃₋₆, C₃₋₉, C₃₋₁₂, C₆₋₉, C₆₋₁₂, and C₉₋₁₂). Similarly, n-membered to (n+m)-membered means that the number of atoms in the ring ranges from n to n+m (e.g., examples of a 3- to 12-membered ring include a 3-membered ring, 4-membered ring, 5-membered ring, 6-membered ring, 7-membered ring, 8-membered ring, 9-membered ring, 10-membered ring, 11-membered ring, and 12-membered ring), as well as any range within n to n+m (e.g., examples of a 3- to-12-membered ring include a 3- to 6-membered ring, 3- to 9-membered ring, 5- to 6-membered ring, 5- to 7-membered ring, 6- to 7-membered ring, 6- to 8-membered ring, and 6- to 10-membered ring).

Unless otherwise specified, the term "C₃₋₇ cycloalkyl" refers to a saturated cyclic hydrocarbon group consisting of 3 to 7 carbon atoms, encompassing monocyclic and bicyclic systems, wherein the bicyclic system encompasses spiro rings, fused rings, and bridged rings. C₃₋₇ cycloalkyl encompasses C₃₋₆ cycloalkyl, C₄₋₆ cycloalkyl, C₄₋₅ cycloalkyl, C₅₋₇ cycloalkyl, C₅₋₆ cycloalkyl, etc., and can be monovalent, divalent, or polyvalent. Examples of C₃₋₇ cycloalkyl include, but are not limited to, cyclopropyl, cyclobutyl, cyclopentyl, cyclohexyl, and cycloheptyl.

Unless otherwise specified, the term "3- to 7-membered heterocycloalkyl," either alone or in combination with other terms, refers to a saturated cyclic group consisting of 3 to 7 ring atoms, of which 1, 2, 3, or 4 ring atoms are heteroatoms independently selected from O, S, and N, with the remaining atoms being carbon. The nitrogen atoms may optionally be quaternized, and the nitrogen and sulfur heteroatoms may optionally be oxidized (i.e., NO and S(O)ₚ, where p is 1 or 2). The term encompasses monocyclic and bicyclic systems, wherein the bicyclic system includes spiro rings, fused rings, and bridged rings. Additionally, with respect to the "3- to 7-membered heterocycloalkyl," a heteroatom may occupy the position connecting the heterocycloalkyl to the rest of the molecule. The 3- to 7-membered heterocycloalkyl encompasses 5- to 7-membered, 3-membered, 4-membered, 5-membered, 6-membered, 7-membered heterocycloalkyl, etc. Examples of 3- to 7-membered heterocycloalkyl include, but are not limited to, azetidinyl, oxetanyl, thietanyl, pyrrolidinyl, pyrazolidinyl, imidazolidinyl, tetrahydrothienyl (including tetrahydrothien-2-yl, tetrahydrothien-3-yl, etc.), tetrahydrofuranyl (including tetrahydrofuran-2-yl, etc.), tetrahydropyranyl, piperidinyl (including 1-piperidinyl, 2-piperidinyl, 3-piperidinyl, etc.), piperazinyl (including 1-piperazinyl, 2-piperazinyl, etc.), morpholinyl (including 3-morpholinyl, 4-morpholinyl, etc.), dioxanyl, dithianyl, isoxazolidinyl, isothiazolidinyl, 1,2-oxazinyl, 1,2-thiazinyl, or hexahydropyridazinyl.

Unless otherwise specified, the term "3- to 7- membered nitrogen-containing heterocycloalkyl" refers to a 3- to 7-membered heterocycloalkyl containing at least one nitrogen atom.

The term "alicyclic group" refers to a saturated or partially unsaturated all-carbon cyclic system, wherein "partially unsaturated" means a ring moiety that includes at least one double or triple bond. The term "partially unsaturated" is intended to encompass rings with multiple unsaturated sites but is not intended to encompass aryl or heteroaryl moieties as defined herein. Non-limiting examples include cyclopropyl ring, cyclobutyl ring, cyclopentyl ring, cyclopentenyl ring, cyclohexyl ring, cyclohexenyl ring, cyclohexadienyl ring, cycloheptyl ring, cycloheptatrienyl ring, cyclopentanone ring, and cyclopentane-1,3-dione ring.

The term "heteroalicyclic group" refers to a saturated or partially unsaturated alicyclic group in which 1, 2, or 3 ring carbon atoms are replaced with heteroatoms selected from nitrogen, oxygen, or S(O)t (where t is an integer from 0 to 2), excluding ring moieties containing -O-O-, -O-S-, or -S-S-, with the remaining ring atoms being carbon. Non-limiting examples include epoxypropane ring, azetidine ring, oxetane ring, tetrahydrofuran ring, tetrahydrothiophene ring, tetrahydropyrrole ring, piperidine ring, pyrroline ring, oxazolidine ring, piperazine ring, dioxolane ring, dioxane ring, morpholine ring, thiomorpholine ring, thiomorpholinyl-1,1-dioxide, tetrahydropyran ring, azetidin-2-one ring, oxetidin-2-one ring, pyrrolidin-2-one ring, pyrrolidin-2,5-dione ring, piperidin-2-one ring, dihydrofuran-2(3H)-one ring, dihydrofuran-2,5-dione ring, tetrahydro-2H-pyran-2-one ring, piperazin-2-one ring, and morpholin-3-one ring. Non-limiting examples of partially unsaturated monocyclic heterocycles include 1,2-dihydroazetidine ring, 1,2-dihydrooxetadiene ring, 2,5-dihydro-1H-pyrrole ring, 2,5-dihydrofuran ring, 2,3-dihydrofuran ring, 2,3-dihydro-1H-pyrrole ring, 3,4-dihydro-2H-pyran ring, 1,2,3,4-tetrahydropyridine ring, 3,6-dihydro-2H-pyran ring, 1,2,3,6-tetrahydropyridine ring, 4,5-dihydro-1H-imidazole ring, 1,4,5,6-tetrahydropyrimidine ring, 3,4,7,8-tetrahydro-2H-1,4,6-oxadiazolidine ring, 1,6-dihydropyrimidine ring, 4,5,6,7-tetrahydro-1H-1,3-diazapine ring, and 2,5,6,7-tetrahydro-1,3,5-oxadiazepine ring.

The terms "aryl" and "aromatic ring" are used interchangeably and refer to an all-carbon monocyclic or fused polycyclic (i.e., rings sharing adjacent pairs of carbon atoms) group with a conjugated π-electron system. This group can be fused with a cycloalkyl, heterocycloalkyl, cycloalkenyl, heterocycloalkenyl, or heteroaryl ring. The term "C₆₋₁₀ aryl" refers to a monocyclic or bicyclic aryl having 6 to 10 carbon atoms. Non-limiting examples of aryl include phenyl and naphthyl.

The terms "heteroaryl" and "heteroaryl ring" are used interchangeably and refer to a monocyclic, bicyclic, or polycyclic 4n+2 aromatic ring system (e.g., having 6 or 10 π electrons shared in a cyclic arrangement) with ring carbon atoms and ring heteroatoms, where each heteroatom is independently selected from nitrogen, oxygen, and sulfur. In the present application, heteroaryl also encompasses ring systems where the above-described heteroaryl ring is fused with one or more cycloalkyl, heterocycloalkyl, cycloalkenyl, heterocycloalkenyl, or aryl rings. The heteroaryl ring can be optionally substituted. The term "5- to 10-membered heteroaryl" refers to a monocyclic or bicyclic heteroaryl having 5 to 10 ring atoms, of which 1, 2, 3, or 4 ring atoms are heteroatoms. The term "5- to 6- membered heteroaryl" refers to a monocyclic heteroaryl having 5 to 6 ring atoms, of which 1, 2, 3, or 4 ring atoms are heteroatoms. Non-limiting examples include thienyl, furanyl, thiazolyl, isothiazolyl, imidazolyl, oxazolyl, pyrrolyl, pyrazolyl, triazolyl, 1,2,3-triazolyl, 1,2,4-triazolyl, 1,2,5-triazolyl, 1,3,4-triazolyl, tetrazolyl, isoxazolyl, oxadiazolyl, 1,2,3-oxadiazolyl, 1,2,4-oxadiazolyl, 1,2,5-oxadiazolyl, 1,3,4-oxadiazolyl, thiadiazolyl, pyridyl, pyridazinyl, pyrimidinyl, pyrazinyl, triazinyl, and tetrazinyl. The term "8- to 10-membered heteroaryl" refers to a bicyclic heteroaryl having 8 to 10 ring atoms, of which 1, 2, 3, or 4 ring atoms are heteroatoms. Non-limiting examples include indolyl, isoindolyl, indazolyl, benzotriazolyl, benzothienyl, isobenzothienyl, benzofuranyl, benzoisofuranyl, benzimidazolyl, benzoxazolyl, benzoisoxazolyl, benzoxadiazolyl, benzothiazolyl, benzoisothiazolyl, benzothiadiazolyl, indazinyl, purinyl, pyrido[3,2-d]pyrimidinyl, pyrido[2,3-d]pyrimidinyl, pyrido[3,4-d]pyrimidinyl, pyrido[4,3-d]pyrimidinyl, 1,8-naphthyridinyl, 1,7-naphthyridinyl, 1,6-naphthyridinyl, 1,5-naphthyridinyl, pteridinyl, quinolinyl, isoquinolinyl, cinnolinyl, quinoxalinyl, phthalazinyl, and quinazolinyl. The term "heteroatom" refers to nitrogen, oxygen, or sulfur. In heteroaryl containing one or more nitrogen atoms, the connection point can be a carbon or nitrogen atom, as long as the valence permits. A heteroaryl bicyclic system may include one or more heteroatoms in one or both rings.

The term "leaving group" refers to a functional group or atom that can be replaced by another functional group or atom through a substitution reaction (e.g., a nucleophilic substitution reaction). For example, representative leaving groups include trifluoromethanesulfonate; chloro, bromo, iodo; sulfonate groups such as methanesulfonate, toluenesulfonate, p-bromobenzenesulfonate, p-toluenesulfonate, etc.; acyloxy groups such acetoxy group and trifluoroacetoxy group.

The term "protecting group" includes, but is not limited to, "amino protecting group," "hydroxy protecting group," or "thiol protecting group". The term "amino protecting group" refers to a protecting group suitable for preventing side reactions at the amino nitrogen site. Representative amino protecting groups include, but are not limited to: formyl; acyl, such as alkanoyl (e.g., acetyl, trichloroacetyl, or trifluoroacetyl); alkoxycarbonyl, such as tert-butoxycarbonyl (Boc); arylmethoxycarbonyl, such as benzyloxycarbonyl (Cbz) and 9-fluorenylmethoxycarbonyl (Fmoc); arylmethyl, such as benzyl (Bn), trityl (Tr), and 1,1-di-(4'-methoxyphenyl)methyl; silyl, such as trimethylsilyl (TMS) and tert-butyldimethylsilyl (TBS). The term "hydroxy protecting group" refers to a protecting group suitable for preventing side reactions of the hydroxyl group. Representative hydroxy protecting groups include, but are not limited to: alkyl, such as methyl, ethyl, and tert-butyl; acyl, such as alkanoyl (e.g., acetyl); arylmethyl, such as benzyl (Bn), p-methoxybenzyl (PMB), 9-fluorenylmethyl (Fm), and diphenylmethyl (benzhydryl, DPM); silyl, such as trimethylsilyl (TMS) and tert-butyldimethylsilyl (TBS).

Unless otherwise defined, the phrase "substituents each independently selected from" in the present application means that when more than one hydrogen of a group is replaced by substituents, the substituents can be the same or different, and selected from independent substituents.

The compounds, their pharmaceutically acceptable salts, or their stereoisomers of the present application can typically be formulated with one or more pharmaceutical carriers to form suitable dosage forms for administration. These dosage forms are suitable for oral, rectal, topical, intraoral, and other parenteral administrations (e.g., subcutaneous, intramuscular, intravenous, etc.). For example, the dosage forms suitable for oral administration include capsules, tablets, granules, and syrups. The compounds of the present application contained in these formulations can be in the form of solid powders or granules; solutions or suspensions in aqueous or non-aqueous liquids; or water-in-oil or oil-in-water emulsions, etc. The above-described dosage forms can be prepared from an active compound and one or more carriers or excipients using standard pharmaceutical methods. The carrier needs to be compatible with the active compound or other excipients. For solid formulations, commonly used non-toxic carriers include, but are not limited to, mannitol, lactose, starch, magnesium stearate, cellulose, glucose, sucrose, etc. Carriers for liquid formulations include water, saline, aqueous glucose solution, ethylene glycol, polyethylene glycol, etc. The active compound can form a solution or suspension with the carrier.

The compositions of the present application are formulated, dosed, and administered in a manner consistent with medical practice. The "therapeutically effective amount" of the compound administered is determined by factors such as the specific condition being treated, the individual being treated, the cause of the condition, the target of the drug, and the administration method.

The term "therapeutically effective amount" refers to the amount of a compound of the present application that elicits a biological or medical response in an individual, such as reducing or inhibiting enzyme or protein activity, improving symptoms, alleviating conditions, slowing or delaying disease progression, or preventing disease.

The therapeutically effective amount of a compound of the present application, its pharmaceutically acceptable salt, or its stereoisomer contained in the pharmaceutical composition or medicinal composition of the present application is preferably from 0.1 mg/kg to 5 g/kg (body weight).

The term "patient" refers to an animal, preferably a mammal, and most preferably a human. The term "mammal" refers to warm-blooded vertebrate mammals, including, for example, cats, dogs, rabbits, bears, foxes, wolves, monkeys, deer, mice, pigs, and humans.

The term "treatment" refers to alleviating, delaying progression, attenuating, preventing, or maintaining an existing disease or condition (e.g., cancer). Treatment also includes curing one or more symptoms of the disease or condition, preventing development of the symptoms, or alleviating the symptoms to some extent.

The compounds of the present application can be prepared by using various synthetic methods well known to those skilled in the art, including the specific embodiments below, combinations thereof with other chemical synthesis methods, and equivalent alternatives known to those skilled in the art. Preferred embodiments include, but are not limited to, the examples of the present application.

The structures of the compounds of the present application can be confirmed by conventional methods well known to those skilled in the art. When an absolute configuration of a compound is involved, this absolute configuration can be verified using standard techniques in the art. For example, single-crystal X-ray diffraction (SXRD) can be employed, where diffraction intensity data of a cultivated single crystal can be collected using a Bruker D8 Venture diffractometer with CuKα radiation as the source and φ-scans as the scan method. After collecting the relevant data, the crystal structure can be further analyzed using the direct method (Shelxs97) to confirm the absolute configuration.

The solvents used in the present application are commercially available. The following abbreviations are used in the present application: N₂ represents nitrogen gas; DMSO represents dimethyl sulfoxide; Pd(dppf)Cl₂ represents [1,1'-bis(diphenylphosphino)ferrocene]-palladium(II) dichloride; DIEA represents N,N-diisopropylethylamine; THF represents tetrahydrofuran; EtOAc represents ethyl acetate; FA represents formic acid; TFA represents trifluoroacetic acid; Select-F represents a selective fluorinating reagent; tBuOK represents potassium tert-butoxide; HCl represents hydrochloric acid; MeOH represents methanol.

Compounds are named according to conventional nomenclatures in the art or using ChemDraw^{®} software, and commercially available compounds are named using supplier catalog names.

Unless otherwise defined, all technical and scientific terms used herein have the same meaning as commonly understood by those skilled in the technical field of the present application. The terms used in the specification of the present application are solely for the purpose of describing specific embodiments and are not intended to limit the present application. The term "and/or" as used herein includes any and all combinations of one or more of the associated listed items.

### BRIEF DESCRIPTION OF THE DRAWING

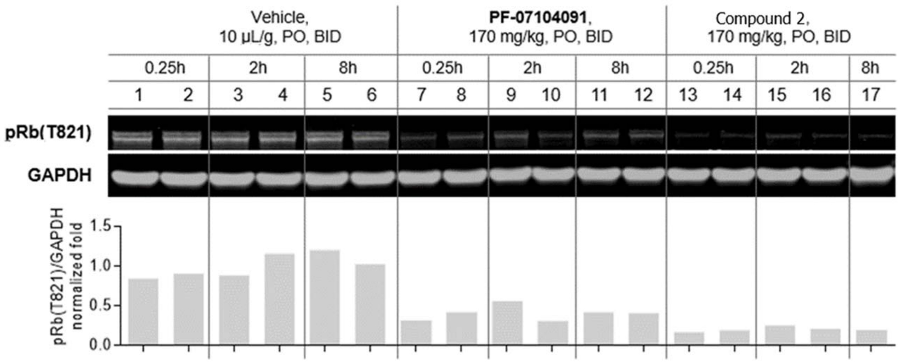

FIG. 1 shows results of Experimental Example 7.

### DETAILED DESCRIPTION

The pyrazole derivative and the preparation method thereof of the present application are further described in detail below with reference to specific examples. Unless otherwise specified, all raw materials used in the following examples are commercially available products.

### Example 1

This example provides compound 1, with the structural formula as follows:

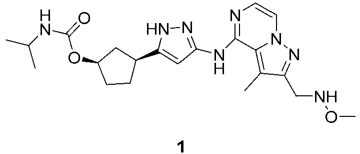

The reaction route is as follows:

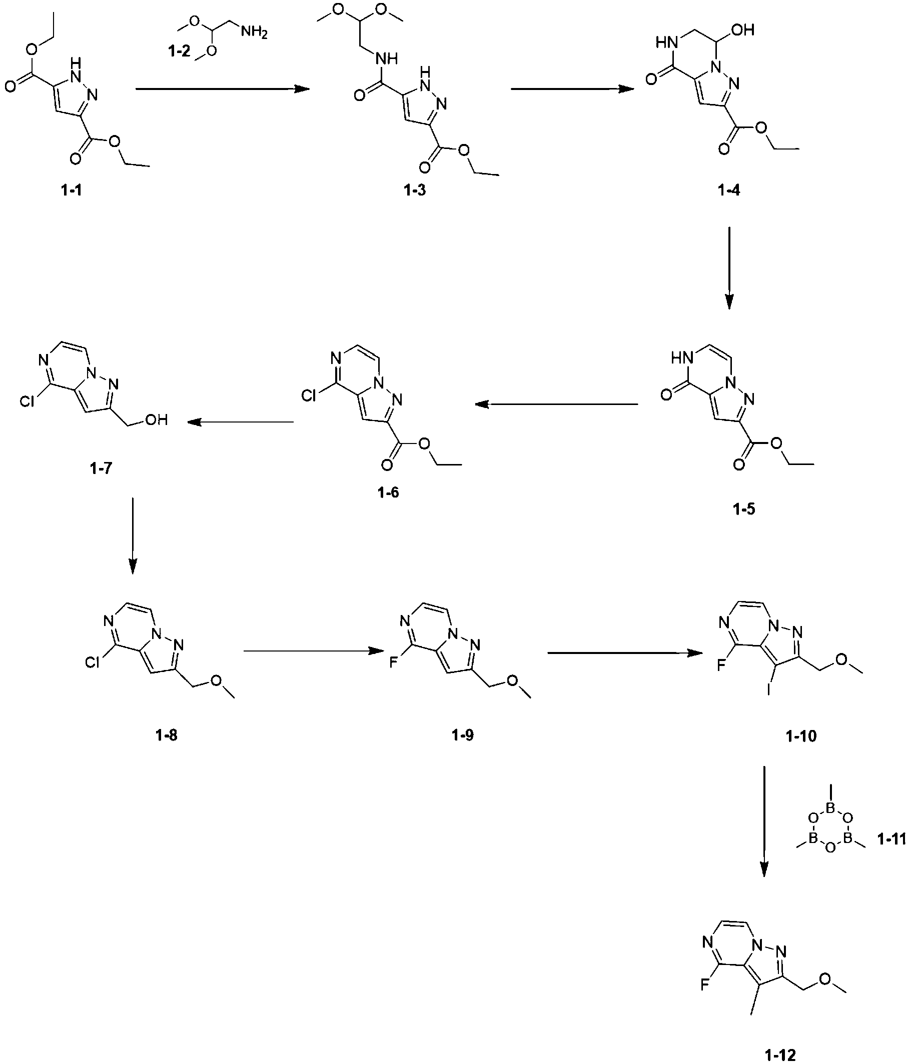

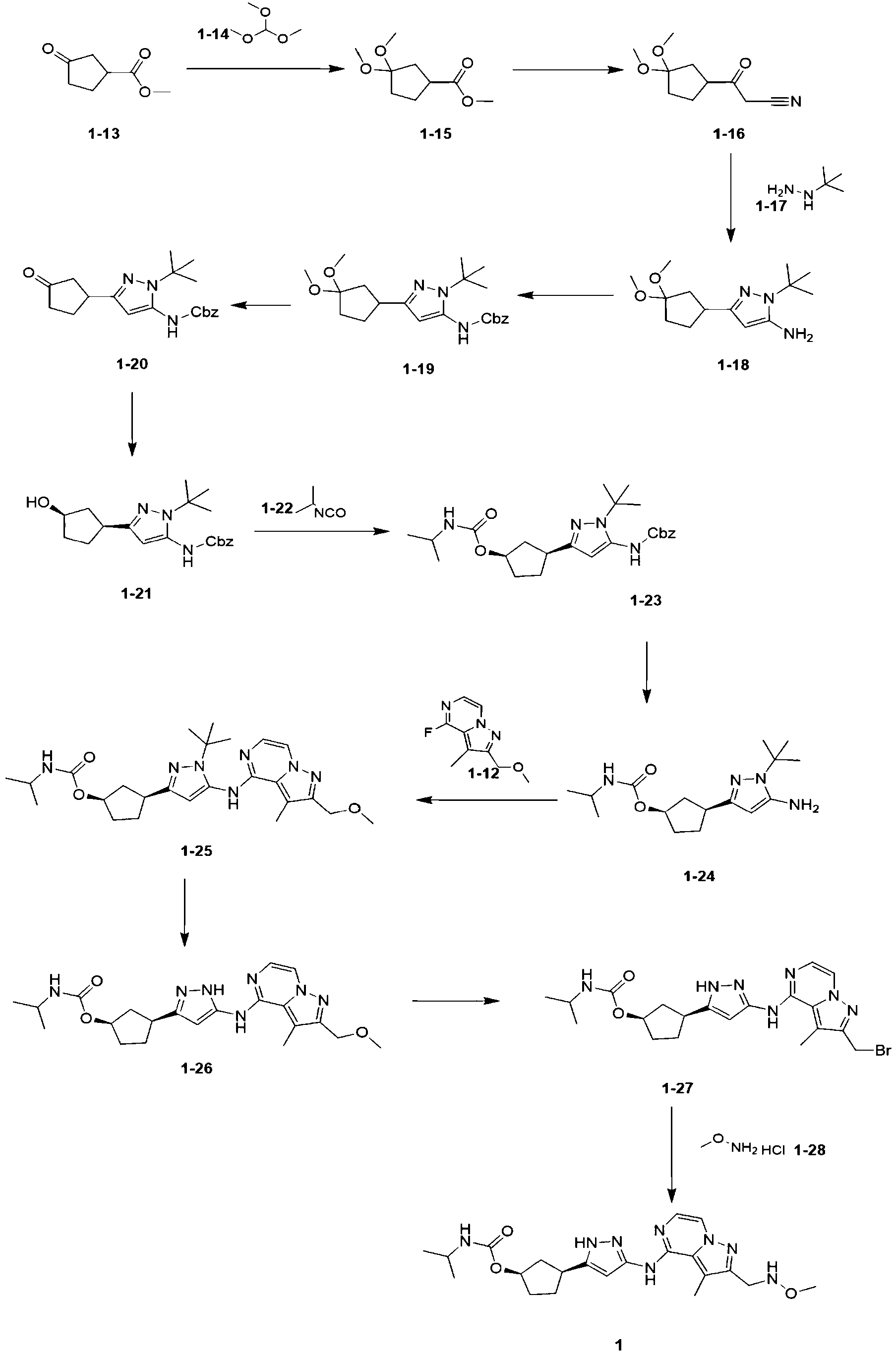

### Step 1:

Compound 1-2 (186 g, 1.77 mol, 192 mL) was dissolved in tetrahydrofuran (700 mL) at room temperature, and cooled to 0 °C. Under N₂ protection, trimethylaluminum solution, 2 M in toluene (2.00 M, 883 mL) was added. After stirring at 25 °C for 1 hour, the reaction system was cooled to 0 °C, and compound 1-1 (250 g, 1.18 mol) dissolved in tetrahydrofuran (500 mL) was added. The air was replaced with N₂ three times, and the reaction was carried out at 25 °C under the N₂ condition for 4 hours. The temperature was adjusted to 0 °C, and methanol (1.00 L) was slowly added dropwise to the reaction system. After reacting at 20 °C for 2 hours, trimethylaluminum was completely quenched. Upon completion of the reaction, the mixture was filtered, and the filtrate was concentrated to obtain a crude product. The crude product was purified by column chromatography (100-200 mesh silica gel, petroleum ether:ethyl acetate = 100:1 to 1:100) to obtain compound 1-3. MS (ESI) m/z = 239.9 [M+H]⁺.

### Step 2:

Compound 1-3 (260 g, 958 mmol) was dissolved in water (500 mL) at room temperature, and cooled to 0 °C. Hydrochloric acid (12 M, 479 mL) was added, and the reaction was carried out at 25 °C for 4 hours. The mixture was filtered, and the filter cake was dried by rotary evaporation to obtain compound 1-4. MS (ESI) m/z = 226.1 [M+H]⁺.

### Step 3:

Compound 1-4 (50.0 g, 222 mmol) was dissolved in methanesulfonic acid (400 g, 4.16 mol, 296 mL) at room temperature, and the reaction was carried out at 25 °C for 10 hours. The reaction solution was slowly poured into a saturated sodium bicarbonate solution (500 mL) and extracted with ethyl acetate (200 mL × 6) six times. The organic phases were combined, dried over anhydrous sodium sulfate, filtered, and the filtrate was concentrated to obtain compound 1-5. MS (ESI) m/z = 207.7 [M+H]⁺.

### Step 4:

Compound 1-5 (11.0 g, 53.1 mmol) and N,N-diisopropylethylamine (6.86 g, 53.1 mmol, 9.25 mL) were dissolved in toluene (100 mL) at room temperature, and cooled to 0 °C. Under N₂ protection, phosphoryl chloride (16.3 g, 106 mmol, 9.87 mL) was added. The mixture was then stirred at 120 °C for 10 hours, followed by concentrating to remove excess toluene and extracting with ethyl acetate (100 mL × 3) three times. The organic phases were combined, washed with saturated brine (20 mL × 2) twice, dried over anhydrous sodium sulfate, filtered, and the filtrate was concentrated to obtain a crude product. The crude product was purified by column chromatography (100-200 mesh silica gel, petroleum ether:ethyl acetate = 100:1 to 1:100) to obtain compound 1-6. MS (ESI) m/z = 225.7 [M+H]⁺. ¹H NMR: DMSO-d6, 400 MHz, δ ppm 8.94 (dd, J = 4.8, 0.80 Hz, 1H), 7.92 (d, J = 4.75 Hz, 1H), 7.46 (d, J = 0.80 Hz, 1H), 4.39 (q, J = 7.2 Hz, 2H), 1.35 (t, J = 7.13 Hz, 3H).

### Step 5:

Compound 1-6 was dissolved in tetrahydrofuran (100 mL), and the air was replaced with N₂ three times. The system was cooled to -68 °C, and diisobutylaluminum hydride (1 M, 75.3 mL) was slowly added in the N₂ atmosphere. The reaction was carried out at 25 °C for 1 hour. At 0 °C, methanol (40.0 mL) was added to the reaction solution, stirred at 25 °C for 2 hours, dried over anhydrous sodium sulfate, and filtered. The filtrate was concentrated to obtain compound 1-7. MS (ESI) m/z = 183.7 [M+H]⁺. ¹H NMR: DMSO-d6, 400 MHz δ ppm 8.77 (d, J = 4.8 Hz, 1H), 7.72 (d, J = 4.4 Hz, 1H), 6.92 (s, 1H), 4.70 (d, J = 6.0 Hz, 2H).

### Step 6:

Compound 1-7 was dissolved in N,N-dimethylformamide (50.0 mL), and the air was replaced with N₂ three times, followed by cooling to -5 °C. In the N₂ atmosphere, methyl iodide (24.5 g, 173 mmol, 10.7 mL) and sodium hydride (1.27 g, 31.7 mmol, 60% purity) were sequentially added to the reaction solution. The reaction was carried out at -5 °C for 1 hour, and then the reaction solution was slowly poured into a saturated ammonium chloride solution (30.0 mL) and extracted with ethyl acetate (20.0 mL × 3) three times. The organic phases were combined, washed with saturated brine (20.0 mL) once, dried over anhydrous sodium sulfate, filtered, and the filtrate was concentrated to obtain compound 1-8. MS (ESI) m/z = 197.7 [M+H]⁺. ¹H NMR: DMSO-d6, 400 MHz δ ppm 8.81 (d, J = 4.8 Hz, 1 H), 7.75 (d, J = 4.4 Hz, 1H), 6.99 (s, 1H), 4.64 (s, 2H), 3.34 (s, 3H).

### Step 7:

Tetrabutylammonium fluoride (21.7 g, 83.3 mmol) was dissolved in N-methylpyrrolidone (25 mL) at room temperature, and the temperature was raised to 130 °C to react for 1 hour. Under N₂ protection, compound 1-8 (5.49 g, 27.7 mmol) was added. The reaction solution was stirred at 130 °C for 3 hours, then poured into ice water (200 mL) and extracted with ethyl acetate (50 mL × 3) three times. The organic phases were combined, washed with saturated brine (30 mL × 2) twice, dried over anhydrous sodium sulfate, filtered, and the filtrate was concentrated to obtain a crude product. The crude product was purified by column chromatography (100-200 mesh silica gel, petroleum ether:ethyl acetate = 100:1 to 1:100) to obtain compound 1-9.

MS (ESI) m/z = 181.7 [M+H]⁺. ¹H NMR: CDCl₃, 400 MHz δ ppm 8.24 (d, J=4.8 Hz, 1H), 7.41 (d, J=4.4 Hz, 1H), 6.88 (s, 1H), 4.72 (s, 2H), 3.48 (s, 3H).

### Step 8:

Compound 1-9 (1.00 g, 5.52 mmol) and N-iodosuccinimide (2.48 g, 11.0 mmol) were dissolved in acetonitrile (10.0 mL) at room temperature, and stirred at 25 °C for 20 hours. The mixture was poured into water (10.0 mL) and extracted with ethyl acetate (10.0 mL × 3) three times. The organic phases were combined, washed with saturated brine (5.00 mL × 2) twice, dried over anhydrous sodium sulfate, filtered, and the filtrate was concentrated to obtain compound 1-10. MS (ESI) m/z = 307.8 [M+H]⁺.

### Step 9:

Compound 1-10 (3.20 g, 10.4 mmol) was dissolved in 1,4-dioxane (20.0 mL), and trimethylboroxine (50% in tetrahydrofuran, 3.5 M, 29.7 mL) and potassium carbonate (4.32 g, 31.2 mmol) were sequentially added to the system. The air was replaced with N₂ three times, and Pd(dppf)Cl₂ (762 mg, 1.04 mmol) was added in the N₂ atmosphere. The reaction was carried out at 90 °C for 10 hours. The reaction solution was poured into water (20.0 mL) and extracted with ethyl acetate (10.0 mL × 3) three times. The organic phases were combined, washed with saturated brine (10.0 mL × 2) twice, dried over anhydrous sodium sulfate, filtered, and the filtrate was concentrated to obtain a crude product. The crude product was purified by column chromatography (100-200 mesh silica gel, petroleum ether:ethyl acetate = 100:1 to 1:100) to obtain compound 1-12. MS (ESI) m/z = 196.1 [M+H]⁺.

### Step 10:

Compound 1-13 (250 g, 1.76 mol) was dissolved in anhydrous methanol (1.00 L), and trimethyl orthoformate (1.31 kg, 12.3 mol, 1.35 L) and p-toluenesulfonic acid (6.06 g, 35.2 mmol) were sequentially added. The reaction was carried out at 25 °C for 30 hours. The reaction solution was slowly poured into a saturated sodium bicarbonate aqueous solution (300 mL), concentrated to remove excess methanol, and extracted with ethyl acetate (200 mL × 2) twice. The organic phases were combined, washed with saturated brine (100 mL × 2) twice, dried over anhydrous sodium sulfate, filtered, and the filtrate was concentrated to obtain compound 1-15.

### Step 11:

Acetonitrile (139 g, 3.40 mol, 179 mL) was dissolved in tetrahydrofuran (700 mL) at room temperature, and the solution was cooled to -68 °C. Under N₂ protection, n-butyllithium (2.5 M in n-hexane, 1.36 L) was added, and the mixture was stirred at -68 °C for 1 hour. Compound 1-15 (320 g, 1.70 mol) dissolved in tetrahydrofuran (300 mL) was then added. The air was replaced with N₂ three times, and the reaction was carried out at -68 °C under the N₂ condition for 1 hour. The reaction solution was slowly poured into ice water (2.00 L), and pH was adjusted to 7 with 1 M hydrochloric acid aqueous solution, and then the reaction solution was extracted with ethyl acetate (1.50 L × 3) three times. The organic phases were combined, washed with saturated sodium chloride aqueous solution (700 mL × 2) twice, dried over anhydrous sodium sulfate, filtered, and the filtrate was concentrated to obtain compound 1-16. ¹H NMR: CDCl₃, 400 MHz δ ppm 3.52 (s, 2H), 3.20 (d, J = 2.40 Hz, 6H), 2.08 (d, J = 8.40 Hz, 3H), 1.97 - 2.04 (m, 1H), 1.83 - 1.92 (m, 3H).

### Step 12:

At room temperature, sodium hydroxide (73.0 g, 1.83 mol) was dissolved in ethanol (500 mL), and tert-butylhydrazine hydrochloride (227 g, 1.83 mol) was added under N₂ protection. The mixture was stirred at 25 °C for 1 hour, and compound 1-16 (320 g, 1.70 mol) dissolved in ethanol (250 mL) was added. The air was replaced with N₂ three times, and the reaction was carried out at 75 °C under the N₂ condition for 15 hours. The reaction solution was filtered, and the filtrate was concentrated to obtain compound 1-18.

### Step 13:

At room temperature, compound 1-18 (425 g, 1.59 mol) was dissolved in acetonitrile (1.00 L), and benzyl chloroformate (542 g, 3.18 mol, 452 mL) was added under N₂ protection. The mixture was stirred at 25 °C for 2 hours, and sodium bicarbonate (401 g, 4.77 mol, 185 mL) was added to the reaction system. The air was replaced with N₂ three times, and the reaction was carried out at 25 °C under the N₂ condition for 11 hours. The reaction mixture was filtered, and the filtrate was concentrated under reduced pressure to obtain compound 1-19. MS (ESI) m/z = 356.1 [M+H]⁺.

### Step 14:

At room temperature, compound 1-19 (758 g, 1.89 mol) and p-toluenesulfonic acid (39.0 g, 226 mmol) were dissolved in water (1.00 L) and acetone (1.00 L). The air was replaced with N₂ three times, and stirring was carried out at 60 °C for 10 hours. The reaction solution was concentrated to remove excess acetone and extracted with dichloromethane (300 mL × 3) three times. The organic phases were combined, washed with saturated brine (200 mL × 2) twice, dried over anhydrous sodium sulfate, filtered, and the filtrate was concentrated to obtain a crude product. The crude product was purified by column chromatography (100-200 mesh silica gel, petroleum ether:ethyl acetate = 100:1 to 1:100) to obtain compound 1-20. MS (ESI) m/z = 356.0 [M+H]⁺.

### Step 15:

Compound 1-20 (177 g, 497 mmol) was dissolved in tetrahydrofuran (900 mL) at room temperature, and cooled to -68 °C. Under N₂ protection, 1 M lithium triethylborohydride solution in tetrahydrofuran (1 M, 996 mL) was added, and stirred at -68 °C for 1.5 hours. The temperature was adjusted to -30 °C, and a saturated sodium bicarbonate aqueous solution (900 mL) was slowly added dropwise to the reaction system. The temperature was further adjusted to -10 °C, and hydrogen peroxide (362 g, 3.20 mol, 307 mL, 30% purity) was slowly added dropwise to the reaction system. After reacting at 10 °C for 1 hour, 1 M lithium triethylborohydride solution in tetrahydrofuran was completely quenched. Upon completion of the reaction, the mixture was extracted with ethyl acetate (1000 mL × 3) three times. The organic phases were combined and slowly poured into a stirred saturated sodium sulfite solution, and care was taken to manage exothermic reactions. The oxidizing property was tested with moist starch-potassium iodide test paper to ensure that the paper did not turn blue. The organic phase was then washed twice with a saturated sodium bicarbonate aqueous solution, followed by washing with saturated brine, dried over anhydrous sodium sulfate, filtered, and concentrated to obtain a crude product. The crude product was extracted with ethyl acetate (1.00 L × 3) three times. The organic phases were combined, washed with saturated sodium chloride aqueous solution (700 mL × 2) twice, dried over anhydrous sodium sulfate, filtered, and the filtrate was concentrated to obtain a crude product. The crude product was purified by column chromatography (100-200 mesh silica gel, petroleum ether:ethyl acetate = 100:1 to 1:100) to obtain the target product. The product was subjected to SFC (column: DAICEL CHIRALPAK AD (250 mm × 30 mm, 10 µm); mobile phase: [CO₂-MeOH]; B%: 35%, isocratic elution) to obtain compound 1-21.

Compound 1-21 was subject to SFC (column: Chiralpak AD-3 50 mm × 4.6 mm I.D., 3 µm; mobile phase: A: CO₂, B: MeOH (0.05% DEA); elution gradient: 5% to 40% B in A; flow rate: 3 mL/min; detector: PDA; column temperature: 35 °C; back pressure: 100 Bar), RT = 1.158 min.

MS (ESI) m/z = 358.7 [M+H]⁺. ¹H NMR: DMSO-d6, 400 MHz δ ppm 9.06 (s, 1H), 7.27 - 7.46 (m, 5H), 5.92 (s, 1H), 5.12 (s, 2H), 4.56 (d, J = 4.4 Hz, 1H), 4.10 - 4.19 (m, 1H), 2.89 (t, J = 8.0 Hz, 1H), 2.14 - 2.23 (m, 1H), 1.80 - 1.91 (m, 1H), 1.65 - 1.78 (m, 2H), 1.52 - 1.62 (m, 2H), 1.48 (s, 9H), 1.04 (d, J = 6.2 Hz, 1H).

### Step 16:

Compound 1-21 (43.0 g, 120 mmol) was dissolved in tetrahydrofuran (100 mL), and triethylamine (36.5 g, 360 mmol, 50.2 mL) and isopropyl isocyanate (40.9 g, 481 mmol, 47.2 mL) were sequentially added. The reaction was carried out at 80 °C for 10 hours. The reaction solution was slowly poured into water (100 mL) and extracted with dichloromethane (100 mL × 2) twice. The organic phases were combined, washed with saturated brine (50.0 mL × 2) twice, dried over anhydrous sodium sulfate, filtered, and the filtrate was concentrated to obtain a crude product. The crude product was purified by column chromatography (100-200 mesh silica gel, petroleum ether:ethyl acetate = 100:1 to 2:1) to obtain compound 1-23. MS (ESI) m/z = 443.8 [M+H]⁺.

¹H NMR: DMSO-d6, 400 MHzδ ppm 9.07 (s, 1H), 7.23 - 7.46 (m, 5H), 6.92 (d, J =8.0 Hz, 1H), 5.93 (s, 1H), 5.11 (s, 2H), 3.55 - 3.62 (m, 1H), 2.90 - 2.99 (m, 1H), 2.36 (t, J= 16, 8.0 Hz, 1H), 1.89 - 1.97 (m, 1H), 1.84 (td, J= 8.0, 4.4 Hz, 1H), 1.56 - 1.75 (m, 4H), 1.47 (s, 9H), 1.02 (d, J= 6.6 Hz, 6H).

### Step 17:

Compound 1-23 (32.0 g, 72.3 mmol) was dissolved in anhydrous tetrahydrofuran (100 mL), and the air was replaced with N₂ three times. Wet palladium on carbon (4.00 g, 50% purity) was added. The N₂ was replaced with H₂ (15 psi) three times. The reaction was carried out at 25 °C under H₂ protection (15 psi) for 2 hours. The reaction solution was filtered, and the filtrate was concentrated to obtain compound 1-24. MS (ESI) m/z = 309.3 [M+H]⁺.

¹H NMR: CDCl₃, 400 MHz δ ppm 6.90 (d, J = 8.0 Hz, 1H), 5.22 (s, 1H), 4.70 (s, 2H), 3.52 - 3.60 (m, 1H), 2.78 (s, 1H), 2.25 - 2.33 (m, 1H), 1.51 - 1.89 (m, 6H), 1.47 (s, 9H), 1.03 (d, J = 6.4 Hz, 6H).

### Step 18:

Compound 1-24 (1.10 g, 3.57 mmol) was dissolved in tetrahydrofuran (10.0 mL) at room temperature, and cooled to -20 °C. Under N₂ protection, lithium bis(trimethylsilyl)amide solution, 1.0 M in tetrahydrofuran (1.0 M, 10.7 mL, 3.00 eq) was added, and stirred at -20 °C for 1 hour. Compound 1-12 (730 mg, 3.74 mmol) dissolved in tetrahydrofuran (5.00 mL) was then added. The air was replaced with N₂ three times, and the reaction was carried out at -20 °C under the N₂ condition for 1 hour. The reaction solution was poured into a saturated ammonium chloride aqueous solution (10.0 mL) and extracted with ethyl acetate (10.0 mL × 3) three times. The organic phases were combined, washed with saturated brine (5.00 mL × 2) twice, dried over anhydrous sodium sulfate, filtered, and the filtrate was concentrated to obtain compound 1-25. MS (ESI) m/z = 484.2 [M+H]⁺.

### Step 19:

Compound 1-25 (1.00 g, 2.07 mmol) was dissolved in anhydrous formic acid (5.00 mL). The reaction was carried out at 100 °C for 0.5 hours. The reaction solution was concentrated to remove formic acid, yielding compound 1-26. MS (ESI) m/z = 428.2 [M+H]⁺.

### Step 20:

Compound 1-26 (160 mg, 374 µmol) was dissolved in dichloromethane (2.00 mL) at room temperature, and cooled to -70 °C. Under N₂ protection, boron tribromide (187 mg, 748 µmol, 72.1 µL) was added, and stirred at 20 °C for 10 hours. The reaction solution was poured into an ice-water solution (5.00 mL) and extracted with dichloromethane (3.00 mL × 3) three times. The organic phases were combined, washed with saturated brine (3.00 mL × 2) twice, dried over anhydrous sodium sulfate, filtered, and the filtrate was concentrated to obtain compound 1-27. MS (ESI) m/z = 478.0 [M+H]⁺.

### Step 21:

At room temperature, DIEA (157 mg, 1.22 mmol, 212 µL) was added to a solution of compound 1-27 (44.7 mg, 93.9 µmol) and compound 1-28 (44.1 mg, 939 µmol) in THF (1.00 mL), and stirred at 50 °C for 5 hours. The reaction system was diluted with 5.0 mL H₂O and extracted with EtOAc (5.0 mL × 3) three times. The organic phases were washed with saturated sodium chloride solution (5.0 mL × 3) three times, dried over anhydrous sodium sulfate, filtered, and concentrated under reduced pressure to obtain a crude product. The crude product was purified by high-performance liquid chromatography (FA conditions: column: Phenomenex Luna C18 150 mm × 25.0 mm × 10.0 µm; mobile phase: [water (FA)-ACN]; gradient: 46.0%-76.0% B over 10.0 min) to obtain a formate salt of compound 1. MS (ESI) m/z =443.1 [M+H] ⁺. ¹H NMR: DMSO-d6, 400MHz δ = 12.28 - 12.13 (m, 1H), 11.72 - 11.52 (m, 1H), 8.27 (br s, 1H), 8.07 - 7.98 (m, 1H), 7.34 (dd, J = 5.6, 11.8 Hz, 1H), 7.31 - 7.30 (m, 1H), 7.05 - 7.00 (m, 1H), 6.83 - 6.75 (m, 1H), 5.97 - 5.88 (m, 1H), 5.12 - 4.99 (m, 2H), 4.14 - 4.12 (m, 1H), 4.04 (d, J = 6.4 Hz, 1H), 3.71 - 3.57 (m, 2H), 3.41 (s, 4H), 3.21 - 3.09 (m, 2H), 2.60 (s, 3H), 2.13 - 2.08 (m, 1H), 2.00 - 1.95 (m, 1H), 1.71 (br s, 4H), 1.12 - 1.07 (m, 10H).

### Example 2

This example provides compound 2, with the structural formula as follows:

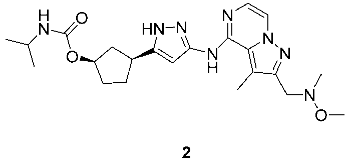

The reaction route is as follows:

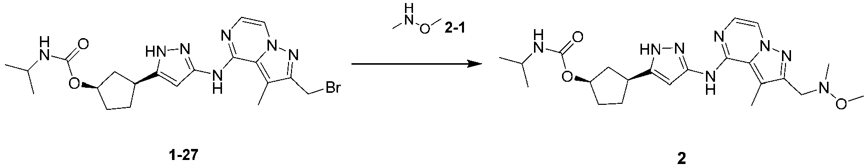

### Step 1:

Compound 2-1 (245 mg, 2.52 mmol) and N,N-diisopropylethylamine (406 mg, 3.15 mmol, 548 µL) were dissolved in tetrahydrofuran (2.00 mL), and the air was replaced with N₂ three times. Compound 1-27 (150 mg, 314 µmol) dissolved in tetrahydrofuran (1.00 mL) was added, and the reaction was carried out at 25 °C for 10 hours. The reaction solution was extracted with ethyl acetate (10.0 mL × 2) three times. The organic phases were combined, washed with saturated brine (10.0 mL × 2) twice, dried over anhydrous sodium sulfate, filtered, and the filtrate was concentrated to obtain a crude product. The crude product was purified by reverse-phase high-performance liquid chromatography (0.1% formic acid condition) to obtain a formate salt of compound 2. MS (ESI) m/z = 457.1 [M+H]⁺. ¹H NMR: DMSO-d6, 400 MHz δ ppm12.18 (br s, 1H), 11.59 (s, 1H), 7.99 (d, J = 4.8 Hz, 1H), 7.29 (dd, J = 5.2, 16.8 Hz, 1H), 7.01 - 6.91 (m, 1H), 5.88 (s, 1H), 5.01 (br s, 1H), 3.93 (s, 1H), 3.84 (s, 1H), 3.58 (br d, J = 6.8 Hz, 1H), 3.31 (s, 3H), 3.09 (brdd, J = 1.6, 6.4 Hz, 1H), 2.56 (s, 3H), 2.53 (br s, 3H), 2.08 - 2.02 (m, 1H), 1.95 - 1.86 (m, 1H), 1.75 - 1.64 (m, 4H), 1.05 - 1.02 (m, 6H).

### Example 3

This example provides compound 3, with the structural formula as follows:

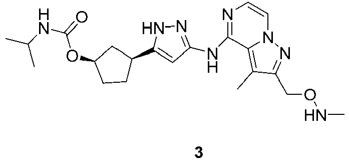

The reaction route is as follows:

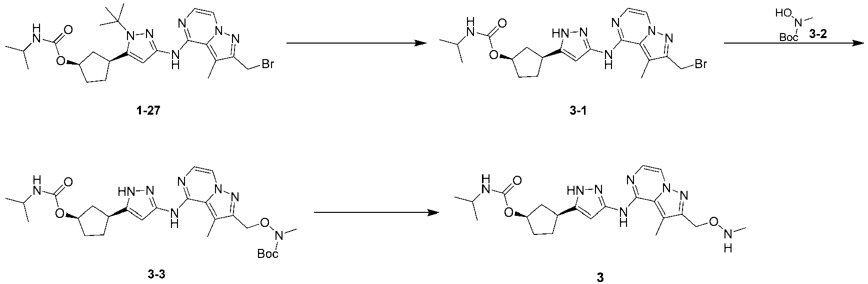

### Step 1:

At room temperature, compound 1-27 (350 mg, 657 µmol) was dissolved in formic acid (3.00 mL). The reaction was carried out at 90 °C for 30 minutes. The reaction solution was directly concentrated to obtain a crude product. The crude product was purified by HPLC (preparative column: Welch Xtimate C18 150 mm × 25 mm × 5 µm; mobile phase: [water(TFA)-ACN]; gradient: 24%-54% B over 10 min) to obtain compound 3-1. MS (ESI) m/z = 478.0 (M+H)⁺.

### Step 2:

Compound 3-2 (154 mg, 1.50 mmol) was dissolved in tetrahydrofuran (1.00 mL) at room temperature, and cooled to 0 °C. Sodium hydride (10.5 mg, 262 µmol, 60% purity) was added, and stirred at 0 °C for 30 minutes. Compound 3-1 (50.0 mg, 52 µmol, TFA salt) was dissolved in tetrahydrofuran (0.50 mL) and slowly added dropwise to the above reaction solution at 0 °C. After the addition was complete, the temperature was raised to 25 °C, and the reaction was carried out for 30 minutes. The reaction solution was poured into ice water (1.00 mL) and extracted with ethyl acetate (1.00 mL × 3) three times. The organic phases were combined, washed with saturated sodium chloride aqueous solution (1.00 mL), dried over anhydrous sodium sulfate, filtered, and the filtrate was concentrated to obtain a crude product. The crude product was purified by HPLC (preparative column: Welch Xtimate C18 150 mm × 25 mm × 5 µm; mobile phase: [water(TFA)-ACN]; gradient: 27%-57% B over 10 min) to obtain compound 3-3. MS (ESI) m/z = 543.7 (M+H)⁺.

### Step 3:

At room temperature, compound 3-3 (28.0 mg, 42.6 µmol, TFA salt) was dissolved in HCl/dioxane (1.00 mL). The reaction was carried out at 25 °C for 30 minutes. The reaction solution was directly concentrated, dissolved in methanol, and dried to obtain a hydrochloride salt of compound 3. m/z = 443.1 (M+H)⁺. ¹H NMR: (400 MHz METHANOL-d4) δ= 8.23 (d, J = 5.6 Hz, 1H), 7.49 (d, J = 5.6 Hz, 1H), 6.40 (s, 1H), 5.45 (s, 2H), 5.12 (br s, 1H), 3.76 - 3.65 (m, 1H), 3.29 - 3.22 (m, 1H), 3.06 (s, 3H), 2.78 (s, 3H), 2.69 - 2.57 (m, 1H), 2.29 - 2.14 (m, 1H), 2.06 - 1.77 (m, 4H), 1.17 - 1.08 (m, 6H).

### Example 4

This example provides compound 4, with the structural formula as follows:

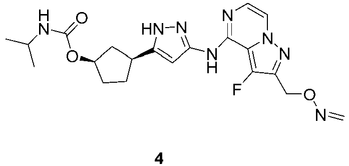

The reaction route is as follows:

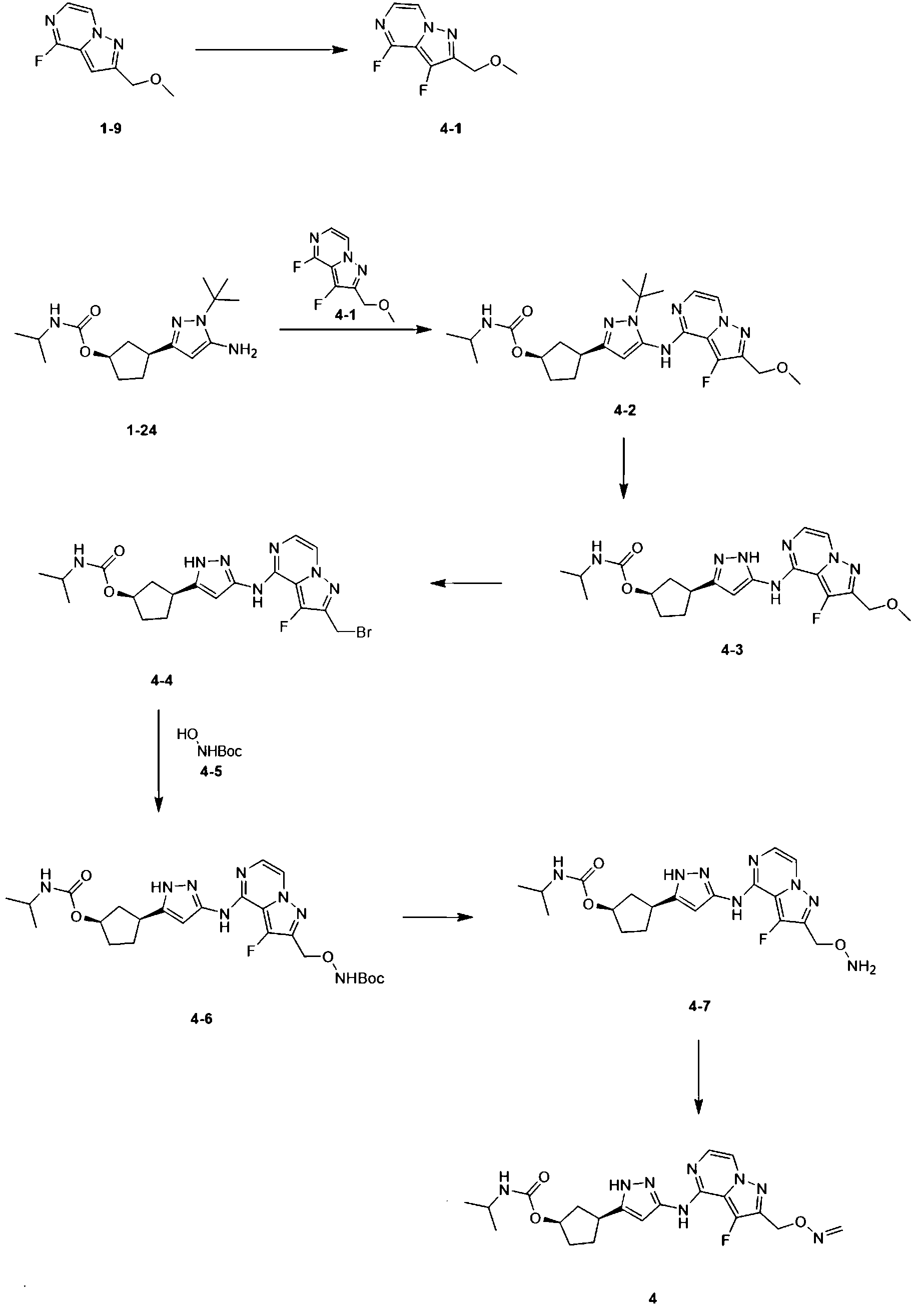

### Step 1:

Compound 1-9 (500 mg, 2.76 mmol) and Select-F (2.93 g, 8.28 mmol) were dissolved in acetonitrile (10.0 mL) at room temperature, and stirred at 25 °C for 20 hours. The reaction solution was poured into water (10.0 mL) and extracted with ethyl acetate (10.0 mL × 3) three times. The organic phases were combined, washed with saturated brine (5.00 mL × 2) twice, dried over anhydrous sodium sulfate, filtered, and the filtrate was concentrated to obtain compound 4-1. MS (ESI) m/z = 199.9 [M+H]⁺.

### Step 2:

Compound 1-24 (800 mg, 2.59 mmol) was dissolved in tetrahydrofuran (8.00 mL) at room temperature, and cooled to -20 °C. Under N₂ protection, lithium bis(trimethylsilyl)amide solution, 1.0 M in tetrahydrofuran (1 M, 7.78 mL) was added, and the mixture was stirred at -20 °C for 1 hour. Compound 4-1 (516 mg, 2.59 mmol) dissolved in tetrahydrofuran (5.00 mL) was then added. The air was replaced with N₂ three times, and the reaction was carried out at -20 °C under the N₂ condition for 1 hour. The reaction solution was poured into a saturated ammonium chloride aqueous solution (10.0 mL) and extracted with ethyl acetate (10.0 mL × 3) three times. The organic phases were combined, washed with saturated brine (5.00 mL × 2) twice, dried over anhydrous sodium sulfate, filtered, and the filtrate was concentrated to obtain compound 4-2. MS (ESI) m/z = 488.1 [M+H]⁺.

### Step 3:

Compound 4-2 (1.12 g, 2.30 mmol) was dissolved in anhydrous formic acid (5.00 mL). The reaction was carried out at 100 °C for 0.5 hours. The reaction solution was concentrated to remove formic acid, yielding a crude product. The crude product was purified by reverse-phase high-performance liquid chromatography (0.1% formic acid condition) to obtain compound 4-3. MS (ESI) m/z = 432.0 [M+H]⁺.

### Step 4:

Compound 4-3 (315 mg, 730 µmol) was dissolved in dichloromethane (3.00 mL) at room temperature, and cooled to -70 °C. Under N₂ protection, boron tribromide (365 mg, 1.46 mmol, 140 µL) was added, and stirred at 20 °C for 10 hours. The reaction solution was poured into an ice-water solution (5.00 mL) and extracted with dichloromethane (3.00 mL × 3) three times. The organic phases were combined, washed with saturated brine (3.00 mL × 2) twice, dried over anhydrous sodium sulfate, filtered, and the filtrate was concentrated to obtain compound 4-4. MS (ESI) m/z = 481.9 [M+H]⁺.

### Step 5:

tBuOK (105 mg, 936 µmol) was added to a solution of compound 4-4 (150 mg, 312 µmol) and compound 4-5 (415 mg, 3.12 mmol) in THF (1.00 mL), and stirred at 0 °C for 10 minutes. The reaction system was diluted with 10.0 mL H₂O and extracted with EtOAc (5.0 mL × 3) three times. The organic phases were washed with saturated sodium chloride solution (5.00 mL × 3) three times, dried over anhydrous sodium sulfate, filtered, and concentrated under reduced pressure to obtain the crude product compound 4-6. MS (ESI) m/z =533.2 [M+H]⁺.

### Step 6:

Compound 4-6 (160 mg, 300 µmol) was stirred in HCl/ethyl acetate (1.00 mL) for 1 hour. The reaction system was diluted with 10.0 mL H₂O and extracted with EtOAc (5.0 mL × 3) three times. The organic phases were washed with saturated sodium chloride solution (5.00 mL × 3) three times, dried over anhydrous sodium sulfate, filtered, and concentrated under reduced pressure to obtain a crude product compound 4-7. MS (ESI) m/z =533.2 [M+H]⁺.

### Step 7:

Polyoxymethylene (90.2 mg, 3.01 mmol, 82.8 µL) was added to a solution of compound 4-7 (130 mg, 300 µmol) in MeOH (0.80 mL) and water (0.20 mL), and stirred at 25 °C for 1 hour. The reaction system was diluted with 10.0 mL H₂O and extracted with EtOAc (5.00 mL × 3) three times. The organic phases were washed with saturated sodium chloride solution (5.00 mL × 3) three times, dried over anhydrous sodium sulfate, filtered, and concentrated under reduced pressure to obtain a crude product. The crude product was purified by high-performance liquid chromatography (FA conditions: column: Phenomenex Luna C18 150 mm × 25 mm × 10 µm; mobile phase: [water(FA)-ACN]; gradient: 21.0%-51.0% B over 10 min) to obtain compound 4. MS (ESI) m/z =445.0 [M+H]⁺. ¹H NMR: DMSO-d6, 400MHz δ = 12.21 - 12.09 (m, 1H), 8.02 - 7.95 (m, 1H), 7.42 - 7.34 (m, 1H), 7.15 - 7.07 (m, 1H), 7.02 - 6.90 (m, 1H), 6.65 (br d, J = 7.2 Hz, 1H), 5.28 - 5.18 (m, 2H), 5.17 - 4.95 (m, 2H), 4.92 - 4.92 (m, 1H), 3.63 - 3.52 (m, 1H), 3.30 (br s, 1H), 3.13 - 3.04 (m, 2H), 2.08 - 2.01 (m, 1H), 1.94 - 1.87 (m, 1H), 1.79 - 1.60 (m, 4H), 1.03 (br d, J = 5.2 Hz, 6H).

### Example 5

This example provides compound 5, with the structural formula as follows:

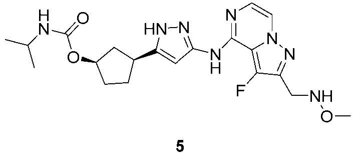

The reaction route is as follows:

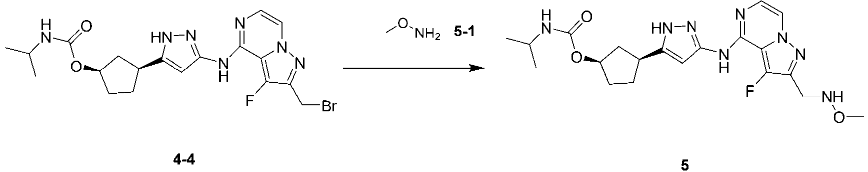

### Step 1:

Compound 5-1 (139 mg, 1.67 mmol, 126 µL) and N,N-diisopropylethylamine (565 mg, 4.37 mmol, 761 µL) were dissolved in tetrahydrofuran (2.00 mL), and the air was replaced with N₂ three times. Compound 4-4 (100 mg, 208 µmol) dissolved in tetrahydrofuran (1.00 mL) was added, and the reaction was carried out at 25 °C for 10 hours. The reaction solution was quenched by pouring into ice water (5.00 mL) and extracted with ethyl acetate (5.00 mL × 2) three times. The organic phases were combined, washed with saturated brine (5.00 mL × 2) twice, dried over anhydrous sodium sulfate, filtered, and the filtrate was concentrated to obtain a crude product. The crude product was purified by reverse-phase high-performance liquid chromatography (0.1% formic acid condition) to obtain compound 5. MS (ESI) m/z = 447.2 [M+H]⁺. ¹H NMR: DMSO-d6, 400 MHz δ ppm12.24 - 12.12 (m, 1H), 8.61 - 8.43 (m, 1H), 7.96 (br d, J = 7.0 Hz, 1H), 7.39 - 7.28 (m, 1H), 7.00 - 6.93 (m, 1H), 6.91 - 6.88 (m, 1H), 6.53 - 6.42 (m, 1H), 5.01 (br s, 1H), 4.09 (d, J = 6.0 Hz, 2H), 3.61 - 3.54 (m, 1H), 3.36 (s, 3H), 3.11 - 3.04 (m, 1H), 2.07 - 2.00 (m, 1H), 1.95 - 1.86 (m, 1H), 1.80 - 1.68 (m, 4H), 1.05 - 1.01 (m, 6H).

### Example 6

This example provides compound 6, with the structural formula as follows:

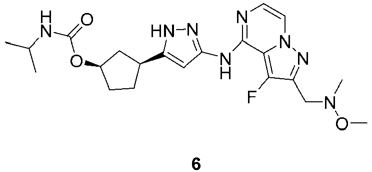

The reaction route is as follows:

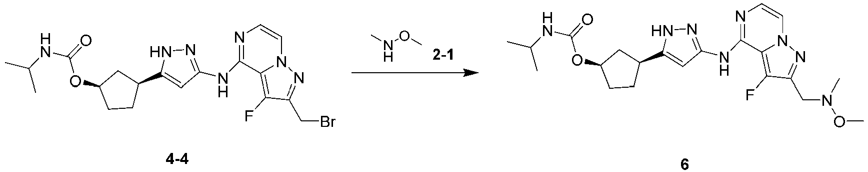

### Step 1:

Compound 2-1 (162 mg, 1.67 mmol) and N,N-diisopropylethylamine (269 mg, 2.08 mmol, 362 µL) were dissolved in tetrahydrofuran (2.00 mL), and the air was replaced with N₂ three times. Compound 4-4 (100 mg, 208 µmol) dissolved in tetrahydrofuran (1.00 mL) was added, and the reaction was carried out at 25 °C for 10 hours. The reaction solution was quenched by pouring into ice water (5.00 mL) and extracted with ethyl acetate (5.00 mL × 2) three times. The organic phases were combined, washed with saturated brine (5.00 mL × 2) twice, dried over anhydrous sodium sulfate, filtered, and the filtrate was concentrated to obtain a crude product. The crude product was purified by reverse-phase high-performance liquid chromatography (0.1% formic acid condition) to obtain compound 6. MS (ESI) m/z = 461.1 [M+H]⁺. ¹H NMR: DMSO-d6, 400 MHz δ ppm 12.24 - 12.09 (m, 1H), 8.59 - 8.45 (m, 1H), 8.02 - 7.92 (m, 1H), 7.34 (br d, J = 4.8 Hz, 1H), 6.96 (br d, J = 8.4 Hz, 1H), 6.49 (br s, 1H), 5.07 - 4.92 (m, 1H), 3.98 (s, 2H), 3.58 (br dd, J = 6.8, 12.0 Hz, 1H), 3.33 - 3.33 (m, 3H), 3.10 (br s, 1H), 2.60 - 2.55 (m, 3H), 2.11 - 1.99 (m, 1H), 1.96 - 1.85 (m, 1H), 1.80 - 1.60 (m, 4H), 1.03 (dd, J = 2.4, 6.4 Hz, 6H).

### Example 7

This example provides compound 7, with the structural formula as follows:

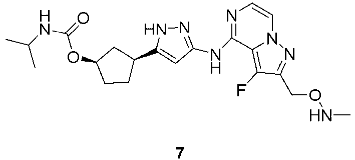

The reaction route is as follows:

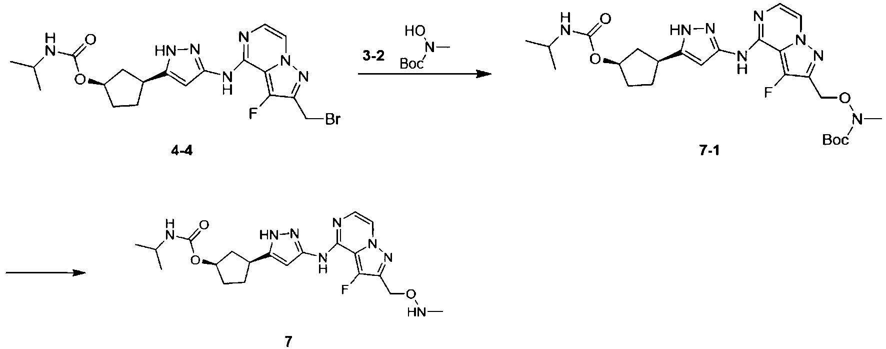

### Step 1:

Compound 3-2 (367 mg, 2.50 mmol) was dissolved in tetrahydrofuran (1.00 mL) at room temperature, and the air was replaced with N₂ three times. The mixture was cooled to 0 °C, and sodium hydride (14.9 mg, 374 µmol, 60% purity) was added and stirred for 30 minutes. Then, compound 4-4 (60.0 mg, 124 µmol) was dissolved in tetrahydrofuran (1.00 mL) and slowly added dropwise to the above reaction solution. The temperature was raised to 20 °C, and the reaction was carried out for 30 minutes. The reaction solution was poured into ice water (1.00 mL) and extracted with ethyl acetate (1.00 mL × 3) three times. The organic phases were combined, washed with saturated sodium chloride aqueous solution (1.00 mL), dried over anhydrous sodium sulfate, filtered, and the filtrate was concentrated to obtain a crude product. The crude product was purified by HPLC (preparative column: Welch Xtimate C18 150 mm × 25 mm × 5 µm; mobile phase: [water(FA)-ACN]; gradient: 32.0%-62.0% B over 10 min) to obtain compound 7-1. m/z = 547.5 (M+H)⁺.

### Step 2:

At room temperature, compound 7-1 (35.0 mg, 59.0 µmol) was dissolved in HCl/EtOAc (0.50 mL). The reaction was carried out at 25 °C for 30 minutes. The reaction solution was directly concentrated, dissolved in methanol, diluted with deionized water, and lyophilized to obtain a hydrochloride salt of compound 7. m/z = 447.0 (M+H)⁺. ¹H NMR: (400 MHz METHANOL-d4) δ8.17 (d, J = 4.8 Hz, 1H), 7.52 (d, J = 5.6 Hz, 1H), 6.34 (s, 1H), 5.40 (s, 2H), 5.12 (br s, 1H), 3.75 - 3.65 (m, 1H), 3.03 (s, 3H), 2.68 - 2.57 (m, 1H), 2.25 - 2.16 (m, 1H), 2.11 - 1.88 (m, 4H), 1.82 (br d, J = 8.4 Hz, 1H), 1.14 - 1.10 (m, 6H).

### In Vitro Activity Testing

### Experimental Example 1: In Vitro CDK2/cyclinE Enzyme Activity Assay

### Experimental Materials:

CDK2/cyclin E1 was purchased from SignalChem. Ulight-4E-BP1 peptide, Eu-anti-phospho-tyrosine antibody, and 1X detection buffer solution were purchased from PerkinElmer. High-purity adenosine triphosphate (ATP) was purchased from Promega. Ethylenediaminetetraacetic acid (EDTA) was purchased from Sigma. Nivo multimode plate reader (PerkinElmer) was used.

### Experimental Methods:

Preparation of Kinase Buffer Solution: The kinase buffer solution consisted 50 mM HEPES, 1 mM EDTA, 10 mM MgCl₂, and 0.01% Brij-35, with pH of 7.4. 2.38 g HEPES, 58 mg EDTA, 406 mg MgCl₂, and 20 mg Brij-35 were added to 200 mL buffer, and the pH was adjusted to 7.4.

### Preparation of Stop Solution:

The stop solution was prepared by mixing 100 µL of 1 M EDTA stock solution with 0.625 µL of 1X detection buffer solution and 1725 µL of distilled water.

The enzyme, Ulight-4E-BP1 peptide, ATP, and the inhibitor were diluted using the kinase buffer solution. The Eu-anti-phospho-tyrosine antibody was diluted to a concentration of 8 nM/L using the detection buffer solution. The test compound was serially diluted 5-fold using a multichannel pipette to the 8^{th} concentration, i.e., from 4 µM to 0.0512 nM. The final concentration of DMSO was 4%, and the experiment was set up with duplicate wells. 2.5 µL of each gradient concentration of the inhibitor, 5 µL of CDK2/cyclin E1 enzyme (10 ng), and 2.5 µL of a mixture of substrate and ATP (4 mM ATP, 100 nM Ulight-4E-BP1 peptide) were added to a microplate, resulting in a final concentration gradient of the compound from 1 µM to 0.0128 nM, with final ATP and substrate concentrations of 1 mM and 25 nM, respectively. The reaction system was incubated at 25 °C for 120 minutes. After the reaction, 5 µL of the stop solution was added to each well, and further reacted at 25 °C for 5 minutes to terminate the reaction. Then 5 µL of Eu-anti-phospho-tyrosine antibody dilution was added to each well, and the mixture was reacted at 25 °C for 60 minutes. Data were collected using the PerkinElmer Nivo multimode plate reader in TR-FRET mode (excitation wavelength: 320 nm; emission wavelengths: 615 nm and 665 nm).

### Data Analysis:

The raw data were converted to inhibition rates according to the equation (Sample-Min)/(Max-Min)×100%. The IC₅₀ value was obtained by fitting the data to a four-parameter curve (using log(inhibitor) vs. response -- Variable slope model in GraphPad Prism). Table 1 provides the enzyme inhibitory activity of the compounds of the present application against CDK2/cyclin E1.

### Experimental Example 2: In Vitro CDK1/cyclin B1 Enzyme Activity Assay

### Experimental Materials:

CDK1/cyclin B1 was purchased from Carna Biosciences. Ulight-4E-BP1 peptide, Eu-anti-phospho-tyrosine antibody, and 1X detection buffer were purchased from PerkinElmer. High-purity ATP was purchased from Promega. EDTA was purchased from Sigma. Nivo multimode plate reader (PerkinElmer) was used.

### Experimental Methods:

Preparation of Kinase Buffer Solution: The kinase buffer solution consisted 50 mM HEPES, 1 mM EDTA, 10 mM MgCl₂, and 0.01% Brij-35, with pH of 7.4. 2.38 g HEPES, 58 mg EDTA, 406 mg MgCl₂, and 20 mg Brij-35 were added to 200 mL buffer, and the pH was adjusted to 7.4.

### Preparation of Stop Solution:

The stop solution was prepared by mixing 100 µL of 1 M EDTA stock solution with 0.625 µL of 1X detection buffer solution and 1725 µL of distilled water.

The enzyme, Ulight-4E-BP1 peptide, ATP, and the inhibitor were diluted using the kinase buffer solution.

The Eu-anti-phospho-tyrosine antibody was diluted to a concentration of 8 nM/L using the detection buffer solution.

The test compound was serially diluted 5-fold using a multichannel pipette to the 8^{th} concentration, i.e., from 4 µM to 0.0512 nM. The final concentration of DMSO was 4%, and the experiment was set up with duplicate wells. 2.5 µL of each gradient concentration of the inhibitor, 5 µL of CDK1/cyclin B1 enzyme (0.5 ng), and 2.5 µL of a mixture of substrate and ATP (4 mM ATP, 200 nM Ulight-4E-BP1 peptide) were added to a microplate, resulting in a final concentration gradient of the compound from 1 µM to 0.0128 nM, with final ATP and substrate concentrations of 1 mM and 50 nM, respectively. The reaction system was incubated at 25 °C for 60 minutes. After the reaction, 5 µL of the stop solution was added to each well, and further reacted at 25 °C for 5 minutes to terminate the reaction. Then 5 µL of Eu-anti-phospho-tyrosine antibody dilution was added to each well, and the mixture was incubated at 25 °C for 60 minutes. Data were collected using the PerkinElmer Nivo multimode plate reader in TR-FRET mode (excitation wavelength: 320 nm; emission wavelengths: 615 nm and 665 nm).

### Data Analysis:

The raw data were converted to inhibition rates using the equation (Sample-Min)/(Max-Min)×100%. The IC₅₀ value was obtained by fitting the data to a four-parameter curve (using log(inhibitor) vs. response -- Variable slope model in GraphPad Prism). Table 1 provides the enzyme inhibitory activity of the compounds of the present application against CDK1/cyclin B1.

### Experimental Example 3: In Vitro OVCAR-3 cell viability Assay

### Experimental Materials:

1640 medium, fetal bovine serum, and penicillin/streptomycin antibiotics were purchased from Wisent. CellTiter-Glo (cell viability chemiluminescence assay reagent) was purchased from Promega. The OVCAR-3 cell line was purchased from Nanjing Cobioer Biosciences Co., Ltd. Envision multimode plate reader (PerkinElmer) was used.

### Experimental Methods:

OVCAR-3 cells were seeded into a white 384-well plate, with 40 µL of cell suspension containing 300 OVCAR-3 cells per well. The cell plate was incubated overnight in a CO₂ incubator.

The test compound was serially diluted 5-fold using a multichannel pipette to the 8^{th} concentration, i.e., from 2000 µM to 0.00512 µM. The experiment was set up with duplicate wells. 78 µL of culture medium was added to an intermediate plate, and 2 µL per well of the gradient-diluted compound was transferred to the corresponding position in the intermediate plate. After mixing, 10 µL per well of the mixture was transferred to the cell plate. The concentration range of the compound transferred to the cell plate was from 10 µM to 0.026 nM. The cell plate was incubated in a CO₂ incubator for 7 days. Another cell plate was prepared, and the signal value was read on the day of compound addition to serve as the maximum value (Max in the equation below) for data analysis. 10 µL per well of CellTiter-Glo reagent was added to the cell plate, and the cell plate was incubated at room temperature for 10 minutes to stabilize the luminescence signal. The signal was read using the multimode plate reader.

10 µL per well of CellTiter-Glo reagent was added to the cell plate, and the cell plate was incubated at room temperature for 10 minutes to stabilize the luminescence signal. The signal was read using the multimode plate reader.

### Data Analysis:

The raw data were converted to inhibition rates using the equation (Sample-Min)/(Max-Min)×100%. The IC₅₀ value was obtained by fitting the data to a four-parameter curve (using log(inhibitor) vs. response -- Variable slope model in GraphPad Prism). Table 1 provides the inhibitory activity of the compounds of the present application against OVCAR-3 cell proliferation.

### Experimental Example 4: In Vitro T-47D Cell viability Assay

### Experimental Materials:

1640 medium, fetal bovine serum, and penicillin/streptomycin antibiotics were purchased from Wisent. CellTiter-Glo (cell viability chemiluminescence assay reagent) was purchased from Promega. The T-47D cell line was purchased from Nanjing Cobioer Biosciences Co., Ltd. Envision multimode plate reader (PerkinElmer) was used.

### Experimental Methods:

T-47D cells were seeded into a white 384-well plate, with 40 µL of cell suspension containing 300 T-47D cells per well. The cell plate was incubated overnight in a CO₂ incubator.

The test compound was serially diluted 5-fold using a multichannel pipette to the 8^{th} concentration, i.e., from 2000 µM to 0.00512 µM. The experiment was set up with duplicate wells. 78 µL of culture medium was added to an intermediate plate, and 2 µL per well of the gradient-diluted compound was transferred to the corresponding position in the intermediate plate. After mixing, 10 µL per well of the mixture was transferred to the cell plate. The concentration range of the compound transferred to the cell plate was from 10 µM to 0.026 nM. The cell plate was incubated in a CO₂ incubator for 7 days. Another cell plate was prepared, and the signal value was read on the day of compound addition to serve as the maximum value (Max in the equation below) for data analysis. 10 µL per well of CellTiter-Glo reagent was added to the cell plate, and the cell plate was incubated at room temperature for 10 minutes to stabilize the luminescence signal. The signal was read using the Envision multimode plate reader.

10 µL per well of CellTiter-Glo reagent was added to the cell plate, and the cell plate was incubated at room temperature for 10 minutes to stabilize the luminescence signal. The signal was read using the Envision multimode plate reader.

### Data Analysis:

The raw data were converted to inhibition rates using the equation (Sample-Min)/(Max-Min)×100%. The IC₅₀ value was obtained by fitting the data to a four-parameter curve (using log(inhibitor) vs. response -- Variable slope model in GraphPad Prism). Table 1 provides the inhibitory activity of the compounds of the present application against T-47D cell proliferation.

**Table 1**

| Test Compound | CDK2 IC₅₀ (nM) | CDK1 IC₅₀ (nM) | OVCAR-3 cells IC₅₀ (nM) | T-47D cells IC₅₀ (nM) |
|---|---|---|---|---|
| PF-07104091 | 3.1 | 521 | 172 | 377 |
| Formate salt of compound 1 | 3.8 | >1000 | 90 | 293 |
| Formate salt of compound 2 | 4.1 | >1000 | 124 | 465 |
| Hydrochloride salt of compound 3 | 4.2 | >1000 | 187 | 383 |
| Compound 4 | 7.6 | >1000 | 75 | 239 |
| Compound 5 | 6.8 | >1000 | 309 | 193 |
| Compound 6 | 7.4 | >1000 | 104 | 384 |
| Hydrochloride salt of Compound 7 | 9.5 | >1000 | 140 | 492 |

Results and Conclusion: The compounds of the present application exhibit good *in vitro* activity and high selectivity for CDK2 over CDK1. Specifically, IC₅₀ of the formate salt of compound 1 against CDK1 was 1908 nM; IC₅₀ of the formate salt of compound 2 against CDK1 was 4084 nM; IC₅₀ of the hydrochloride salt of compound 3 against CDK1 was 1354 nM; IC₅₀ of compound 5 against CDK1 was 1501 nM; IC₅₀ of compound 6 against CDK1 was 3493 nM; and IC₅₀ of the hydrochloride salt of compound 7 against CDK1 was greater than 10,000 nM, demonstrating superior selectivity compared to PF-07104091.

### In Vivo Pharmacokinetic Study

### Experimental Example 5: Pharmacokinetic Study of Test Compounds Administered Orally and Intravenously in Mice

### Experimental Objective:

This study used ICR male mice as test animals. The plasma concentrations of the test compounds and the reference compound in mice were quantitatively determined at different time points using the LC-MS/MS method following intravenous injection or oral administration to evaluate the pharmacokinetic characteristics of the test compounds in mice.

### Experimental Materials:

ICR mice (male, 20-30 g, 6-8 weeks old, Beijing Charles River).

### Experimental Procedure:

A clear solution of the test compound was administered to ICR mice (fasted overnight or fed) via tail vein injection or oral gavage. For intravenous injection, blood samples (50 µL) were collected via cheek puncture at 0 h (pre-dose) and at 0.083, 0.25, 0.5, 1, 2, 4, 6, 8, and 24 h post-dose into anticoagulant tubes containing sodium heparin. The mixtures were thoroughly vortexed at 4 °C and centrifuged at 6000 rpm for 3 minutes. For oral gavage, blood samples were collected via cheek puncture at 0 h (pre-dose) and at 0.083, 0.25, 1, 2, 4, 6, 8, and 24 h post-dose into anticoagulant tubes containing sodium heparin. The mixtures were vortexed thoroughly, and centrifuged at 6000 rpm for 3 minutes. Plasma drug concentrations were determined using the LC-MS/MS method. Pharmacokinetic parameters were calculated using Phoenix WinNonlin 8.2.0 pharmacokinetic software with a non-compartmental model and linear-logarithmic trapezoidal rule. The experimental results are shown in Table 2.

**Table 2**

| Route of administration | Pharmacokinetic parameters | PF-07104091 | Compound 5 | Compound 6 |
|---|---|---|---|---|
| Intravenous administration | Dose (mg/Kg) | 5 | 5 | 5 |
| | Half-life T_{1/2} (h) | 2.68 | 6.48 | 0.93 |
| | Clearance CL (ml/min/Kg) | 50.3 | 13.0 | 6.35 |
| | Apparent volume of distribution Vdss (L/Kg) | 11.8 | 7.58 | 0.52 |
| | Area under plasma concentration-time curve AUC₀₋ₗₐₛₜ (ng.h/mL) | 1645 | 6773 | 13216 |
| Oral administration | Dose (mg/Kg) | 30 | 30 | 30 |
| | Time to peak Tₘₐₓ (h) | 0.25 | 0.19 | 1.08 |
| | Peak concentration Cₘₐₓ (ng/mL) | 6317 | 22100 | 41100 |
| | Area under plasma concentration-time curve AUC₀₋ₗₐₛₜ (ng.h/mL) | 7524 | 52609 | 155301 |
| | Bioavailability F (%) | 76.2 | 129 | 195.8 |

Results and Conclusion: The compounds of the present application demonstrate excellent bioavailability. At the same dose, the time to peak concentration and AUC are significantly better than those of PF-07104091.

### In Vivo Efficacy Study

### Experimental Example 6: In Vivo Pharmacodynamic Study in Human Ovarian Cancer OVCAR-3 Cell Subcutaneous Xenograft Tumor CB17-SCID Mice Model

### Experimental Procedure:

Cell Culture: OVCAR-3 cells (human ovarian cancer cell line, sourced from Medsyin's cell bank) were cultured in RPMI-1640 medium (ATCC modified, Gibco) supplemented with 20% FBS (Gibco) and 1% penicillin/streptomycin (P.S.), *in vitro* at 37 °C with 5% CO₂. The cells were passaged twice weekly. OVCAR-3 cells in the exponential growth phase were collected and resuspended in a mixture of PBS and Matrigel (Corning) to prepare a cell suspension with a concentration of 5×10⁷ cells/mL for mouse inoculation. The volume ratio of PBS to Matrigel was 1:1.

Animals: CB17-SCID mice, female, 6-8 weeks old, weighing 18-21 grams. A total of 40 mice were inoculated. The mice were provided by Shanghai Lingchang Biotechnology Co., Ltd.

Tumor Inoculation: Each mouse was subcutaneously injected in the right flank with 0.2 mL of OVCAR-3 tumor cells (10×10⁶ cells).

Experimental Metrics: The experimental metrics aimed to evaluate whether tumor growth was inhibited, delayed, or cured. The tumor diameter was measured twice weekly using a vernier caliper. The tumor volume was calculated using the formula: V=0.5a×b², where a and b represent the longest and shortest diameters of the tumor, respectively.

Antitumor metric TGI (%) reflects the tumor growth inhibition rate. TGI (%) was calculated as follows: TGI (%) = [(1 - (average tumor volume at the end of drug administration in a given group - average tumor volume at the start of drug administration in that group)) / (average tumor volume at the end of treatment in a vehicle control group - average tumor volume at the start of treatment in the vehicle control group)] × 100%.

**Experimental Results:** The experimental results are shown in Table 3.

**Table 3**

| Compound | Dose | Route of administration | Dosing frequency | TGI |
|---|---|---|---|---|
| Formate salt of compound 2 | 35 mg/Kg | Oral | BID*21 days | 104.1% |
| Formate salt of compound 2 | 70 mg/Kg | Oral | BID*21 days | 114.1% |
| Formate salt of compound 2 | 140 mg/Kg | Oral | BID*21 days | 114.6% |

The compounds of the present application demonstrated unexpected tumor regression effects in the OVCAR-3 *in vivo* efficacy model, with good animal tolerability.

### In Vivo Pharmacodynamic (PD) Study

### Experimental Example 7: In Vivo PD Study in OVCAR-3 Human Ovarian Cancer Mouse Model

### Experimental Procedure:

Cell Culture: OVCAR-3 human ovarian cancer cells (ATCC, Manassas, cat # HTB-161) were cultured in monolayer *in vitro* at 37 °C in a 5% CO₂ incubator. The culture medium was RPMI 1640 supplemented with 20% fetal bovine serum, 0.01 mg/mL bovine insulin, and 1% anti-anti. Cells were passaged twice weekly using trypsin-EDTA for routine digestion. When cell confluency reached 80%-90% and the required cell number was achieved, cells were harvested, counted, and prepared for inoculation.

Animals: BALB/c nude mice, female, 7-9 weeks old, weighing 18-23 grams. A total of 100 mice were inoculated. The mice were provided by the Experimental Animal Division of Shanghai Institute of Planned Parenthood Research (formerly Shanghai SIPPR-BK).

Tumor Inoculation: Each mouse was subcutaneously inoculated with 0.2 mL (10×10⁶ cells) of OVCAR-3 cells (mixed with Matrigel in a 1:1 volume ratio) in the right flank. When the average tumor volume reached 171 mm³, the mice were divided into groups for dosing, with 6 mice per group, across 10 groups.

Tumor Sampling: Tumor samples were collected after 21 days of continuous dosing.

### Protein Extraction and Quantification:

The frozen tissue samples were retrieved from a -80 °C freezer or liquid nitrogen tank.

On dry ice, approximately 30-100 mg of the tissue was taken, placed in a 2 mL centrifuge tube with steel beads, and 400 µL of RIPA lysis buffer (freshly supplemented with 1% protease and phosphatase inhibitors) was added.

The tissue was disrupted using a cryogenic grinder at 50 Hz for 5 minutes.

The tissue lysate was incubated on ice for 30 minutes.

The lysate was centrifuged at 15,000 rpm at 4 °C for 10 minutes, and the supernatant was transferred to a new 1.5 mL centrifuge tube.

Protein quantification was performed using a BCA assay kit.

Based on the quantification results, protein samples for loading were prepared, with the protein concentration standardized to 4 µg/µL. LDS sample buffer (4X) and sample reducing agent (10X) were added, and the samples were heated at 100 °C for 10 minutes.

The denatured samples were either used for Western blotting or stored at -80 °C.

### Western Blotting:

Loading: The samples were thawed and loaded, 10 µL per well in a Bis-Tris gel.

Electrophoresis: Electrophoresis was performed in 1X MES electrophoresis buffer solution at 80 V for 30 minutes, followed by 120 V for 90 minutes.

Transfer: Transfer was performed using the iBlot 2 transfer kit and transfer device, with a program set at 20 V for 7 minutes.

After the transfer, the membrane was cut according to the molecular weight of the target protein and washed with 10 mL 1X TBS three times, 5 minutes each, at room temperature with shaking.

Blocking: The membrane was blocked in 10 mL of Intercept^{®} (TBS) blocking buffer at room temperature with gentle shaking for 1 hour.

Primary Antibody Incubation: 10 mL of appropriately diluted primary antibodies (diluted in Intercept^{®} (TBS) blocking buffer containing 0.1% Tween-20) was added, and the membrane was incubated at 4 °C with gentle shaking, wherein the pRb (T821) antibody was diluted at 1:1000 and incubated for 3 days, while the GAPDH antibody was diluted at 1:2000 and incubated overnight.

The membrane was washed with 10 mL 1X TBST three times, 10 minutes each, at room temperature with shaking.

Secondary Antibody Incubation: 10 mL of appropriately diluted secondary antibodies (diluted at 1:10,000 in Intercept^{®} (TBS) blocking buffer containing 0.1% Tween-20) was added, and the membrane was incubated at room temperature with gentle shaking for 1 hour.

The membrane was washed with 10 mL 1X TBST three times, 10 minutes each, at room temperature with shaking.

The membrane was washed with 10 mL 1X TBS twice, 5 minutes each, at room temperature with shaking to remove residual Tween-20.

Fluorescent signals were detected using the Odyssey Clx and its Image Studio Ver 5.2 software.

### Expression Quantification:

Relative quantification of the Western blot fluorescence bands was performed using Image Studio Lite Ver 5.2 software.

### Data Analysis:

Band intensities obtained from the software quantification were analyzed to calculate the expression ratio of the target protein relative to the reference protein (GAPDH). The normalized fold expression of the target protein in the dosing groups was calculated based on the blank control group as reference. The normalized fold expressions of the protein across groups were presented in GraphPad Prism 6.02.

### Experimental Results: The results are shown in FIG. 1.

**Experimental Conclusion:** According to the Western blot experiment, the compounds of the present application can effectively inhibit CDK2 enzyme activity, reduce the level of pRb protein, and thereby block the transition from G1 phase to S phase of the cell cycle, inhibiting tumor cell proliferation. At the same dose, the compounds of the present application exhibited superior inhibitory activity against CDK2 downstream pRb phosphorylated at T821 compared to PF-07104091.

The technical features of the above-mentioned embodiments can be combined arbitrarily. In order to make the description concise, not all possible combinations of the technical features are described in the embodiments. However, as long as there is no contradiction in the combination of these technical features, the combinations should be considered as in the scope of the present application.

The above-described embodiments are only several implementations of the present application for specifically and comprehensively understanding the technical solution of the present application, but they should not be construed as limiting the scope of the present application. It should be understood by those of ordinary skill in the art that various modifications and improvements can be made without departing from the concept of the present application, and all fall within the protection scope of the present application. It should be understood that the technical solutions obtained by those skilled in the art, on the basis of the technical solution provided by the present application, through logical analysis, reasoning or limited experiments, are all within the protection scope of the appended claims of the present application. Therefore, the patent protection scope of the present application shall be defined by the appended claims, and the specification can be used to explain the content of the claims.

## Claims

1. A pyrazole derivative represented by Formula (I), or a pharmaceutically acceptable salt thereof, or a stereoisomer thereof: 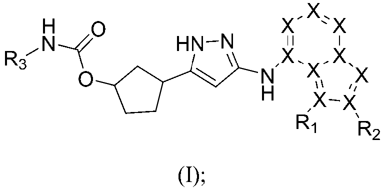 wherein:
X, at each occurrence, is independently selected from N or CR₄; R₄ is absent or selected from -H, -D, -F, -Cl, -Br, -I, linear alkyl having 1 to 6 carbon atoms, deuterated linear alkyl having 1 to 6 carbon atoms, branched alkyl having 3 to 6 carbon atoms, or an aromatic heterocyclic group;
R₁ is selected from -H, -D, -F, -Cl, -Br, -I, linear alkyl having 1 to 20 carbon atoms, deuterated linear alkyl having 1 to 20 carbon atoms, branched alkyl having 3 to 20 carbon atoms, deuterated branched alkyl having 3 to 20 carbon atoms, cycloalkyl having 3 to 20 carbon atoms, deuterated cycloalkyl having 3 to 20 carbon atoms, linear alkoxy having 1 to 20 carbon atoms, deuterated linear alkoxy having 1 to 20 carbon atoms, branched alkoxy having 3 to 20 carbon atoms, deuterated branched alkoxy having 3 to 20 carbon atoms, cycloalkoxy having 3 to 20 carbon atoms, or deuterated cycloalkoxy having 3 to 20 carbon atoms;
R₃ is selected from substituted or unsubstituted alkyl having 1 to 6 carbon atoms, substituted or unsubstituted fluoroalkyl having 1 to 6 carbon atoms, substituted or unsubstituted cycloalkyl having 3 to 7 carbon atoms, or a substituted or unsubstituted heterocyclic group having 4 to 7 ring atoms;
R₂ is selected from -F, -Cl, -OH, -CN, -NR₅R₆, -CH₂-ONR₇R₈, -CH₂-ON=R₉, -CH₂N(R₁₀)OR₁₁, substituted or unsubstituted alkyl having 1 to 4 carbon atoms, substituted or unsubstituted fluoroalkyl having 1 to 4 carbon atoms, substituted or unsubstituted alkoxy having 1 to 4 carbon atoms, substituted or unsubstituted fluoroalkoxy having 1 to 4 carbon atoms, or substituted or unsubstituted cycloalkyl having 3 to 8 carbon atoms;
R₅, R₆, R₇, R₈, R₁₀, and R₁₁ are each independently selected from -H, -D, linear alkyl having 1 to 20 carbon atoms, deuterated linear alkyl having 1 to 20 carbon atoms, branched alkyl having 3 to 20 carbon atoms, deuterated branched alkyl having 3 to 20 carbon atoms, cycloalkyl having 3 to 20 carbon atoms, deuterated cycloalkyl having 3 to 20 carbon atoms, linear alkoxy having 1 to 20 carbon atoms, deuterated linear alkoxy having 1 to 20 carbon atoms, branched alkoxy having 3 to 20 carbon atoms, deuterated branched alkoxy having 3 to 20 carbon atoms, cycloalkoxy having 3 to 20 carbon atoms, or deuterated cycloalkoxy having 3 to 20 carbon atoms;
R₉ is selected from linear alkylidene having 1 to 20 carbon atoms, deuterated linear alkylidene having 1 to 20 carbon atoms, branched alkylidene having 3 to 20 carbon atoms, deuterated branched alkylidene having 3 to 20 carbon atoms, cycloalkylidene having 3 to 20 carbon atoms, or deuterated cycloalkylidene having 3 to 20 carbon atoms.

2. The pyrazole derivative, or the pharmaceutically acceptable salt thereof, or the stereoisomer thereof according to claim 1, wherein the pyrazole derivative is represented by Formula (II):

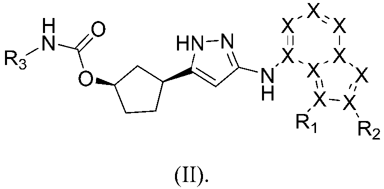

3. The pyrazole derivative, or the pharmaceutically acceptable salt thereof, or the stereoisomer thereof according to claim 2, wherein the pyrazole derivative is represented by Formula (III):

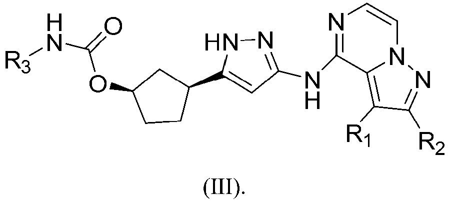

4. The pyrazole derivative, or the pharmaceutically acceptable salt thereof, or the stereoisomer thereof according to claim 1, wherein R₁ is selected from -H, -D, -F, -Cl, -Br, -I, linear alkyl having 1 to 10 carbon atoms, deuterated linear alkyl having 1 to 10 carbon atoms, branched alkyl having 3 to 10 carbon atoms, deuterated branched alkyl having 3 to 10 carbon atoms, cycloalkyl having 3 to 10 carbon atoms, or deuterated cycloalkyl having 3 to 10 carbon atoms.

5. The pyrazole derivative, or the pharmaceutically acceptable salt thereof, or the stereoisomer thereof according to claim 1, wherein R₅, R₆, R₇, R₈, R₁₀, and R₁₁ are each independently selected from -H, -D, linear alkyl having 1 to 10 carbon atoms, deuterated linear alkyl having 1 to 10 carbon atoms, branched alkyl having 3 to 10 carbon atoms, deuterated branched alkyl having 3 to 10 carbon atoms, cycloalkyl having 3 to 10 carbon atoms, deuterated cycloalkyl having 3 to 10 carbon atoms, linear alkoxy having 1 to 10 carbon atoms, deuterated linear alkoxy having 1 to 10 carbon atoms, branched alkoxy having 3 to 10 carbon atoms, deuterated branched alkoxy having 3 to 10 carbon atoms, cycloalkoxy having 3 to 10 carbon atoms, or deuterated cycloalkoxy having 3 to 10 carbon atoms;
R₉ is selected from linear alkylidene having 1 to 20 carbon atoms, deuterated linear alkylidene having 1 to 20 carbon atoms, branched alkylidene having 3 to 20 carbon atoms, deuterated branched alkylidene having 3 to 20 carbon atoms, cycloalkylidene having 3 to 20 carbon atoms, deuterated cycloalkylidene having 3 to 20 carbon atoms, or combinations thereof.

6. The pyrazole derivative, or the pharmaceutically acceptable salt thereof, or the stereoisomer thereof according to claim 1, wherein R₂ is any one selected from following structures:

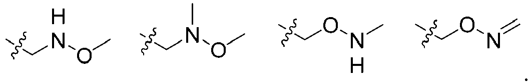

7. The pyrazole derivative, or the pharmaceutically acceptable salt thereof, or the stereoisomer thereof according to any one of claims 1 to 6, wherein the pyrazole derivative is any one of following compounds:

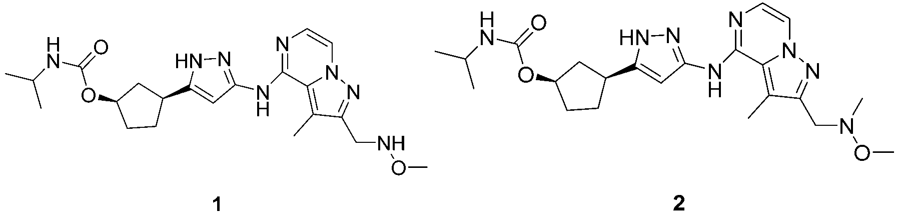

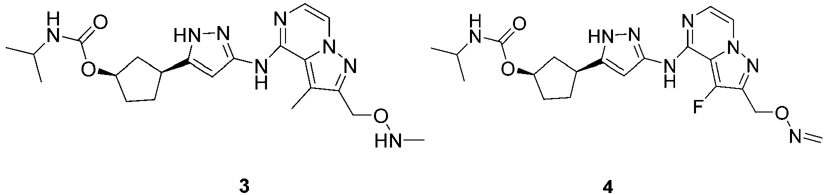

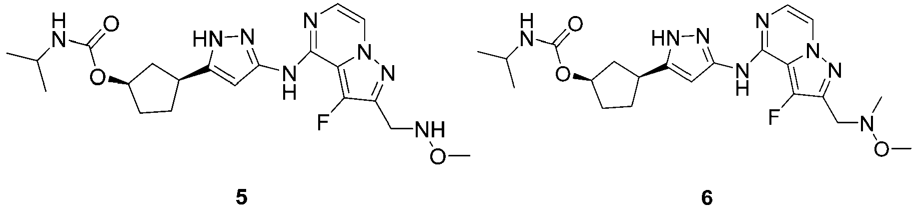

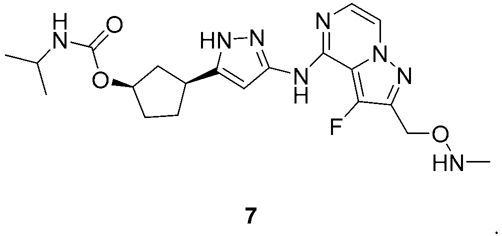

8. Use of the pyrazole derivative, or the pharmaceutically acceptable salt thereof, or the stereoisomer thereof according to any one of claims 1 to 7, in preparation of a drug for treating and/or preventing a disease related to or mediated by CDK2 activity.

9. The use according to claim 8, wherein the disease related to or mediated by CDK2 activity is cancer.

10. A pharmaceutical composition comprising the pyrazole derivative, or the pharmaceutically acceptable salt thereof, or the stereoisomer thereof according to any one of claims 1 to 7, and a pharmaceutically acceptable carrier.
